# EUROPEAN PATENT APPLICATION

(11) **EP 4 796 545 A1**
(43) Date of publication of application: **26.08.2026**
(21) Application number: 24884103.3
(22) Date of filing: 08.08.2024
(51) Int. Cl.: C07D 495/04, C09K 19/34, C09K 19/44, C09K 19/02, C09K 19/16, C09K 19/18, G02F 1/13, G02F 1/133

(54) **COMPOUNDS, PREPARATION METHOD THEREFOR, AND USE THEREOF**

(30) Priority: 30.10.2023 CN 202311424905
(71) Applicant: HUAWEI TECHNOLOGIES CO., LTD., Shenzhen 518129 (CN)
(72) Inventor: TANG, Yongquan, Shenzhen, Guangdong 518129 (CN); XIANG, Jiaxiang, Shenzhen, Guangdong 518129 (CN); WU, Liang, Shenzhen, Guangdong 518129 (CN); CHEN, Hongyu, Shenzhen, Guangdong 518129 (CN)
(74) Representative: Thun, Clemens
(86) International application number: PCT/CN2024/110795
(87) International publication number: WO 2025/092096

(57) **Abstract**

Provided are a compound, and a preparation method and use thereof, which relate to the field of organic materials. The compound includes a first chain hydrocarbon group, a first phenyl group, a second phenyl group, a thieno[3,2-b]thiophene group, and a second chain hydrocarbon group. The first chain hydrocarbon group is linked to one carbon atom of the first phenyl group, another carbon atom of the first phenyl group is linked to one carbon atom of the second phenyl group by a first linkage bond, another carbon atom of the second phenyl group is linked to one carbon atom of the thieno[3,2-b]thiophene group by a second linkage group, and another carbon atom of the thieno[3,2-b]thiophene group is linked to the second chain hydrocarbon group. The first phenyl group has one or two first substituents, where the first substituent is F, -CF₃, or -OCF₃; and the first chain hydrocarbon group and the second chain hydrocarbon group are linear alkyl, alkoxy, fluoroalkyl, alkenyl, alkenyloxy, or difluorovinyl. The compound can be used as a liquid crystal monomer to improve birefringence, low-temperature stability, high-temperature stability, and a nematic-phase temperature range of a liquid crystal material.

## Description

This application claims priority to Chinese Patent Application No. 202311424905.7, filed on October 30, 2023 and entitled "COMPOUND, AND PREPARATION METHOD AND USE THEREOF", which is incorporated herein by reference in its entirety.

### TECHNICAL FIELD

The present disclosure relates to the field of organic materials, and in particular, to a compound, and a preparation method and use thereof.

### BACKGROUND

A liquid crystal material is usually formed by combining and formulating a plurality of liquid crystal monomers, so that the liquid crystal material is at least characterized by: high birefringence, a wide nematic-phase temperature range, strong temperature resistance (low-temperature stability and high-temperature stability), and an excellent ultraviolet resistance capability.

However, some existing high-birefringence liquid crystal materials, despite their high birefringence, exhibit a weak UV resistance capability, poor high-temperature stability, or a narrow nematic-phase temperature range (-10°C to 110°C), leading to poor low-temperature stability.

### SUMMARY

In view of this, the present disclosure provides a compound, and a preparation method and use thereof, so that technical problems in the conventional technology can be resolved. Specifically, the following technical solutions are included.

An aspect provides a compound. The compound includes a first chain hydrocarbon group, a first phenyl group, a second phenyl group, a thieno[3,2-b]thiophene group, and a second chain hydrocarbon group;
the first chain hydrocarbon group is linked to one carbon atom of the first phenyl group, another carbon atom of the first phenyl group is linked to one carbon atom of the second phenyl group by a first linkage bond, another carbon atom of the second phenyl group is linked to one carbon atom of the thieno[3,2-b]thiophene group by a second linkage group, and another carbon atom of the thieno[3,2-b]thiophene group is linked to the second chain hydrocarbon group;
the first phenyl group has one or two first substituents, the first substituent is adjacent to the carbon atom that is in the first phenyl group and that is linked to the first linkage bond, and the first substituent is F, -CF₃, or -OCF₃; and
the first chain hydrocarbon group and the second chain hydrocarbon group are selected from linear alkyl, linear alkoxy, linear fluoroalkyl, linear alkenyl, linear alkenyloxy, or difluorovinyl.

The compound provided in embodiments of the present disclosure includes the first chain hydrocarbon group, the first phenyl group, the second phenyl group, the thieno[3,2-b]thiophene group, and the second chain hydrocarbon group that are sequentially linked, so that the compound can be used as a liquid crystal monomer. The synergistic effect of the foregoing groups allows the compound to exhibit high birefringence and strong low-temperature stability. In addition, the introduction of the thieno[3,2-b]thiophene group into the compound helps further improve the birefringence of the compound. The first phenyl group has one or two first substituents. The first substituent is F, -CF₃, or -OCF₃, so that ordered arrangement and intermolecular forces of molecules of the compound can be effectively reduced, helping improve low-temperature stability of the compound. When the compound provided in embodiments of the present disclosure is used as a liquid crystal monomer for preparing a liquid crystal material, birefringence, low-temperature stability, and high-temperature stability of a high-birefringence liquid crystal material can be improved. This further helps expand a nematic-phase temperature range of the liquid crystal material.

In some possible implementations, the first chain hydrocarbon group and the second chain hydrocarbon group are each independently selected from the linear alkyl with a number of carbon atoms of 1 to 9, the linear alkoxy with a number of carbon atoms of 1 to 9, the linear fluoroalkyl with a number of carbon atoms of 1 to 9, the linear alkenyl with a number of carbon atoms of 2 to 9, the linear alkenyloxy with a number of carbon atoms of 2 to 9, or the difluorovinyl with a number of carbon atoms of 2 to 9.

In some possible implementations, the second phenyl group is substituted or unsubstituted; and
the substituted second phenyl group has one or more second substituents, and the second substituent is selected from -F, -CH₃, -CH₂CH₃, -OCF₃, cyclopropyl, cyclobutyl, or cyclohexyl.

The second substituent is selected from the foregoing types, helping reduce a melting point of the compound. When the compound provided in embodiments of the present disclosure is used as a liquid crystal monomer for preparing a liquid crystal material, low-temperature stability of a high-birefringence liquid crystal material can be further improved.

In some possible implementations, the first linkage bond and the second linkage bond are each independently selected from a carbon-carbon single bond, a carbon-carbon double bond, an alkynyl bond, an ester bond, or a difluoromethyl ether bridge bond. The foregoing linkage bonds are suitable for improving birefringence of the compound and reducing a crystallization point of the compound.

In some implementations, a chemical structural formula of the compound is shown as follows: 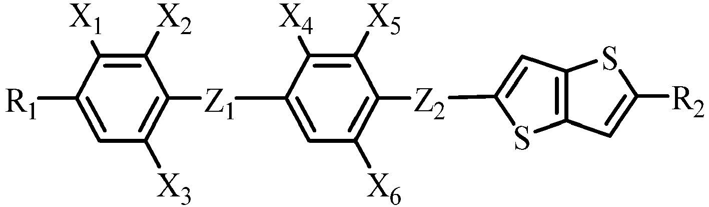
where R₁ and R₂ respectively represent the first chain hydrocarbon group and the second chain hydrocarbon group, and the first chain hydrocarbon group and the second chain hydrocarbon group are each independently selected from the linear alkyl with a number of carbon atoms of 1 to 9, the linear alkoxy with a number of carbon atoms of 1 to 9, the linear fluoroalkyl with a number of carbon atoms of 1 to 9, the linear alkenyl with a number of carbon atoms of 2 to 9, the linear alkenyloxy with a number of carbon atoms of 2 to 9, or the difluorovinyl with a number of carbon atoms of 2 to 9;
Z₁ and Z₂ respectively represent the first linkage bond and the second linkage bond, and the first linkage bond and the second linkage bond are each independently selected from and independently represent a carbon-carbon single bond, a carbon-carbon double bond, alkynyl, ester, or a difluoromethyl ether bridge bond;
X₁ and X₄ to X₆ are each independently selected from -H, -F, -CH₃, -CH₂CH₃, -OCF₃, cyclopropyl, cyclobutyl, or cyclohexyl; and
at least one of X₂ and X₃ is F, -CF₃, or -OCF₃.

The compound of this chemical structure can be used as a liquid crystal monomer. When the compound is used for preparing a liquid crystal material, at least birefringence and low-temperature stability of a high-birefringence liquid crystal material can be improved, and a nematic-phase temperature range of the liquid crystal material can be expanded.

For example, the compound is a compound I-1, a compound I-2, a compound I-3, or a compound I-4; and
a chemical structural formula I-1 corresponding to the compound I-1, a chemical structural formula I-2 corresponding to the compound I-2, a chemical structural formula I-3 corresponding to the compound I-3, and a chemical structural formula I-4 corresponding to the compound I-4 are separately shown as follows: 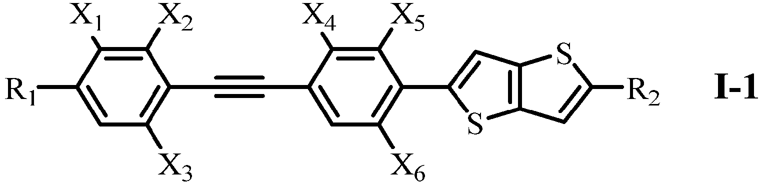 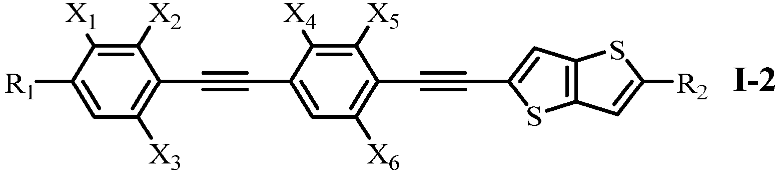 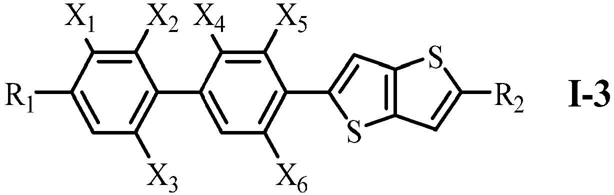 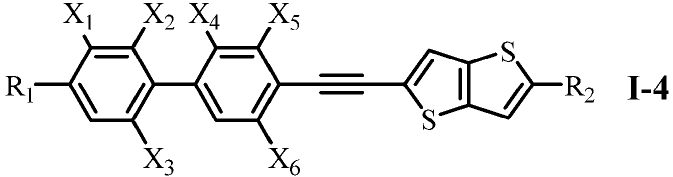
where R₁ and R₂ are each independently selected from the linear alkyl with the number of carbon atoms of 1 to 9 or the linear alkoxy with the number of carbon atoms of 1 to 9.

According to another aspect, an embodiment of the present disclosure provides use of any one of the foregoing compounds in a liquid crystal material.

The compound provided in embodiments of the present disclosure can be used as a liquid crystal monomer, and the compound can be further used with another type of liquid crystal monomer, thereby optimizing the following effects of the liquid crystal material: high birefringence, a wide nematic-phase temperature, excellent low-temperature stability, excellent high-temperature stability, and an excellent ultraviolet resistance capability.

According to still another aspect, an embodiment of the present disclosure provides a method for preparing any one of the foregoing compounds. The preparation method includes:
determining, based on a chemical structure of the compound, a plurality of monomers for synthesizing the compound; and
subjecting the plurality of monomers to a reaction step by step to prepare the compound, where
the reaction includes at least one of a Sonogashira coupling reaction, a Suzuki coupling reaction, and a halogenation reaction.

In embodiments of the present disclosure, the plurality of monomers for synthesizing the compound are determined based on the chemical structure of the compound. For example, a quantity of monomers for synthesizing the compound is at least 4. First, two monomers are subjected to a reaction to form a first intermediate, and then the first intermediate is subjected to a reaction with another monomer to form a second intermediate, and so on. In this case, the compound is finally prepared by using the plurality of monomers through multi-step reactions.

According to still another aspect, an embodiment of the present disclosure provides a liquid crystal material. The liquid crystal material includes a first liquid crystal monomer and a second liquid crystal monomer, and the first liquid crystal monomer is any one of the foregoing compounds. The second liquid crystal monomer is configured to ensure that there is no crystallization phenomenon at a temperature less than or equal to 0°C after the second liquid crystal monomer and the first liquid crystal monomer are mixed.

By using any one of the foregoing compounds provided in embodiments of the present disclosure as a liquid crystal monomer, the liquid crystal material provided in embodiments of the present disclosure exhibits at least high birefringence, a wide nematic-phase temperature, and excellent low-temperature stability. The first liquid crystal monomer and the second liquid crystal monomer are combined. The synergistic effect of the first liquid crystal monomer and the second liquid crystal monomer allows the prepared liquid crystal material to have advantages such as high birefringence, a wide nematic-phase temperature, excellent low-temperature stability, and excellent high-temperature stability.

In some possible implementations, the second liquid crystal monomer is selected from at least one of a compound II-1, a compound II-2, a compound II-3, a compound II-4, a compound II-5, a compound II-6, a compound II-7, and a compound II-8; and
a chemical structural formula II-1 corresponding to the compound II-1, a chemical structural formula II-2 corresponding to the compound II-2, a chemical structural formula II-3 corresponding to the compound II-3, a chemical structural formula II-4 corresponding to the compound II-4, a chemical structural formula II-5 corresponding to the compound II-5, a chemical structural formula II-6 corresponding to the compound II-6, a chemical structural formula II-7 corresponding to the compound II-7, and a chemical structural formula II-8 corresponding to the compound II-8 are separately shown as follows: 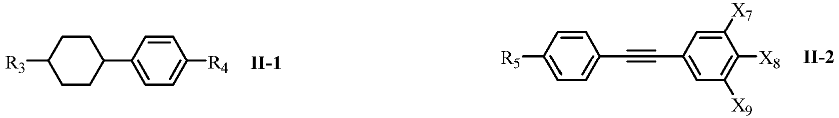 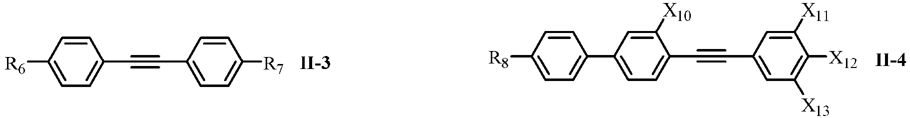 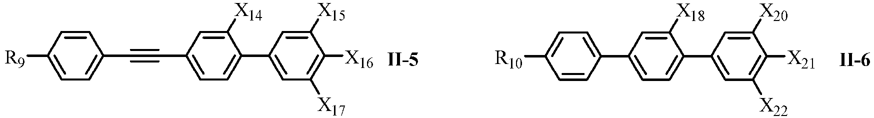 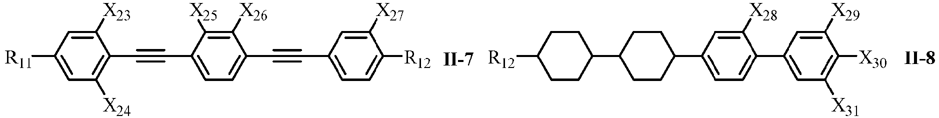
where R₃ to R₁₂ are each independently selected from one of linear alkyl with a number of carbon atoms of 1 to 6 and linear alkoxy with a number of carbon atoms of 1 to 6; and
X₇ to X₃₁ are each independently selected from -H, -F, -CH₃, -CH₂CH₃, or -OCF₃.

In some possible implementations, the liquid crystal material further includes an additive, and the additive is selected from at least one of an ultraviolet absorber, an ultraviolet stabilizer, and an antioxidant.

One or more of the foregoing additives are added to the liquid crystal material, so that an ultraviolet resistance capability and high-temperature stability of the liquid crystal material can be further improved.

In some possible implementations, the liquid crystal material has at least one of the following characteristics: no crystallization at -60°C to 0°C; no color change at 0°C to 150°C; and no color change at a UV intensity less than or equal to 80 mW/cm².

According to still another aspect, an embodiment of the present disclosure further provides an optoelectronic device. The optoelectronic device includes an encapsulation structure and a liquid crystal material encapsulated in the encapsulation structure, and the liquid crystal material is the foregoing liquid crystal material according to embodiments of the present disclosure.

For example, the optoelectronic device includes a wavelength selective switch, a microwave antenna, or a display device.

### DESCRIPTION OF EMBODIMENTS

The following clearly and completely describes technical solutions in embodiments of the present disclosure. It is clear that the described embodiments are merely some rather than all of embodiments of the present disclosure. All other embodiments obtained by a person of ordinary skill in the art based on embodiments of the present disclosure without creative efforts shall fall within the protection scope of the present disclosure.

A liquid crystal material is widely used in the optoelectronic field, for example, used in laser projection, optoelectronic display, and optoelectronic communication. The foregoing application scenarios require the liquid crystal material to be at least characterized by: high birefringence, a wide nematic-phase temperature range, strong temperature resistance (low-temperature stability and high-temperature stability), and an excellent ultraviolet resistance capability.

With no change in optical phase modulation, high birefringence allows for reduction of a thickness of a liquid crystal layer, so that an optoelectronic device can obtain a higher response speed and effectively avoid fringing field effect between pixels of the device, improving optical modulation efficiency. A wider nematic-phase temperature range of the liquid crystal material indicates a wider operating temperature range of the optoelectronic device. To achieve a sufficiently wide nematic-phase temperature range of the liquid crystal material, for example, a crystallization point of the liquid crystal material being less than -20°C (further less than -40°C), and a clearing point of the liquid crystal material being greater than 120°C, a plurality of liquid crystal monomers are required to have good mutual miscibility. A strong ultraviolet resistance capability and strong temperature resistance (for example, high-temperature stability) make the optoelectronic device suitable for an encapsulation condition and an outdoor environment.

However, existing liquid crystal materials, despite their high birefringence, exhibit a weak UV resistance capability, poor high-temperature stability, or a narrow nematic-phase temperature range (-10°C to 110°C), leading to poor low-temperature stability.

In view of the technical problems in the conventional technology, embodiments of the present disclosure provide a compound. The compound includes a first chain hydrocarbon group, a first phenyl group, a second phenyl group, a thieno[3,2-b]thiophene group, and a second chain hydrocarbon group. The first chain hydrocarbon group is linked to one carbon atom of the first phenyl group, another carbon atom of the first phenyl group is linked to one carbon atom of the second phenyl group by a first linkage bond, another carbon atom of the second phenyl group is linked to one carbon atom of the thieno[3,2-b]thiophene group by a second linkage group, and another carbon atom of the thieno[3,2-b]thiophene group is linked to the second chain hydrocarbon group. The first phenyl group has one or two first substituents, the first substituent is adjacent to the carbon atom that is in the first phenyl group and that is linked to the first linkage bond, and the first substituent is F, -CF₃, or -OCF₃; and the first chain hydrocarbon group and the second chain hydrocarbon group are selected from linear alkyl, linear alkoxy, linear fluoroalkyl, linear alkenyl, linear alkenyloxy, or difluorovinyl.

The compound provided in embodiments of the present disclosure includes the first chain hydrocarbon group, the first phenyl group, the second phenyl group, the thieno[3,2-b]thiophene group, and the second chain hydrocarbon group that are sequentially linked, so that the compound can be used as a liquid crystal monomer. The synergistic effect of the foregoing groups allows the compound to exhibit high birefringence and strong low-temperature stability. In addition, the introduction of the thieno[3,2-b]thiophene group into the compound helps further improve the birefringence of the compound. The first phenyl group has one or two first substituents. The first substituent is F, -CF₃, or -OCF₃, so that ordered arrangement and intermolecular forces of molecules of the compound can be effectively reduced, helping improve low-temperature stability of the compound. When the compound provided in embodiments of the present disclosure is used as a liquid crystal monomer for preparing a liquid crystal material, birefringence, low-temperature stability, and high-temperature stability of a high-birefringence liquid crystal material can be improved. This further helps expand a nematic-phase temperature range of the liquid crystal material.

The "high-birefringence liquid crystal material" in embodiments of the present disclosure is a liquid crystal material whose birefringence is greater than 0.3.

In some examples, the first chain hydrocarbon group and the second chain hydrocarbon group are each independently selected from the linear alkyl with a number of carbon atoms of 1 to 9, the linear alkoxy with a number of carbon atoms of 1 to 9, the linear fluoroalkyl with a number of carbon atoms of 1 to 9, the linear alkenyl with a number of carbon atoms of 2 to 9, the linear alkenyloxy with a number of carbon atoms of 2 to 9, or the difluorovinyl with a number of carbon atoms of 2 to 9. The first chain hydrocarbon group and the second chain hydrocarbon group may be the same or may be different.

For example, the linear alkyl with the number of carbon atoms of 1 to 9 includes but is not limited to: methyl, ethyl, propyl, butyl, pentyl, hexyl, and octyl. There may be one, two, or more alkoxy groups in the linear alkoxy with the number of carbon atoms of 1 to 9. In an embodiment of the present disclosure, a chemical formula of the linear alkoxy may be -OCₙH₂ₙ₊₁, including but not limited to: methoxy, ethoxy, propoxy, butoxy, pentyloxy, hexyloxy, and octoxy. There may be one, two, or more fluorine elements in the linear fluoroalkyl.

In an embodiment of the present disclosure, the second phenyl group is substituted or unsubstituted, the substituted second phenyl group has one or more second substituents, and the second phenyl group has four substitutable sites. In an embodiment of the present disclosure, there may be one, two, three, or four second substituents. In a further example, there may be one, two, or three second substituents. When there are a plurality of second substituents, the plurality of second substituents may be the same or may be different.

In some examples, the second substituent is selected from -F, -CH₃, -CH₂CH₃, -OCF₃, cyclopropyl, cyclobutyl, or cyclohexyl.

The second substituent is selected from the foregoing types, helping reduce a melting point of the compound. When the compound provided in embodiments of the present disclosure is used as a liquid crystal monomer for preparing a liquid crystal material, low-temperature stability of a high-birefringence liquid crystal material can be further improved.

In an embodiment of the present disclosure, the first linkage bond and the second linkage bond are each independently selected from a carbon-carbon single bond, a carbon-carbon double bond, an alkynyl bond, an ester bond, or a difluoromethyl ether bridge bond. The foregoing linkage bonds are suitable for improving birefringence of the compound and reducing a crystallization point of the compound.

The first linkage bond and the second linkage bond may be the same or may be different. For example, both the first linkage bond and the second linkage bond are selected from a carbon-carbon single bond, or both the first linkage bond and the second linkage bond are selected from an alkynyl bond, or one of the first linkage bond and the second linkage bond is selected from a carbon-carbon single bond, and the other is selected from an alkynyl bond.

In some possible implementations, a chemical structural formula of the compound is shown as follows: 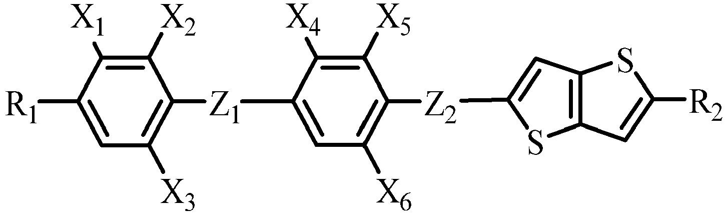
where R₁ and R₂ respectively represent the first chain hydrocarbon group and the second chain hydrocarbon group, and the first chain hydrocarbon group and the second chain hydrocarbon group are each independently selected from the linear alkyl with a number of carbon atoms of 1 to 9, the linear alkoxy with a number of carbon atoms of 1 to 9, the linear fluoroalkyl with a number of carbon atoms of 1 to 9, the linear alkenyl with a number of carbon atoms of 2 to 9, the linear alkenyloxy with a number of carbon atoms of 2 to 9, or the difluorovinyl with a number of carbon atoms of 2 to 9;
Z₁ and Z₂ respectively represent the first linkage bond and the second linkage bond, and the first linkage bond and the second linkage bond are each independently selected from and independently represent a carbon-carbon single bond, a carbon-carbon double bond, alkynyl, ester, or a difluoromethyl ether bridge bond;
X₁ and X₄ to X₆ are each independently selected from -H, -F, -CH₃, -CH₂CH₃, -OCF₃, cyclopropyl, cyclobutyl, or cyclohexyl; and
at least one of X₂ and X₃ is F, -CF₃, or -OCF₃.

The compound of this chemical structure can be used as a liquid crystal monomer. When the compound is used for preparing a liquid crystal material, at least birefringence, low-temperature stability, and high-temperature stability of a high-birefringence liquid crystal material can be improved, and a nematic-phase temperature range of the liquid crystal material can be expanded.

Based on the foregoing chemical structural formula, the compound may be a compound I-1, a compound I-2, a compound I-3, or a compound I-4, where a chemical structural formula I-1 corresponding to the compound I-1, a chemical structural formula I-2 corresponding to the compound I-2, a chemical structural formula I-3 corresponding to the compound I-3, and a chemical structural formula I-4 corresponding to the compound I-4 are separately shown as follows: 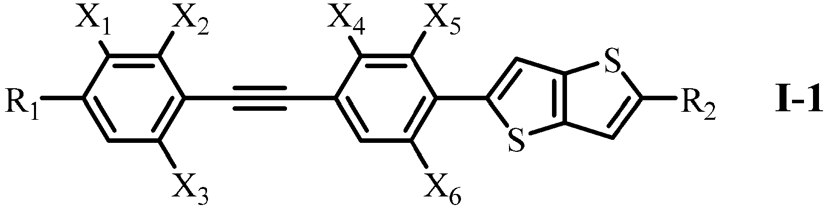 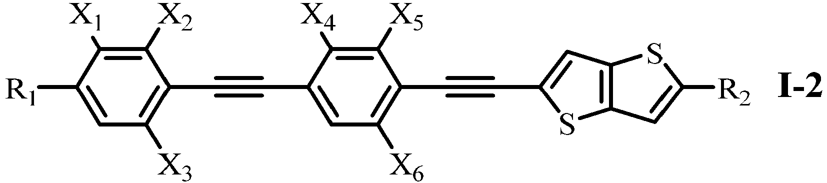 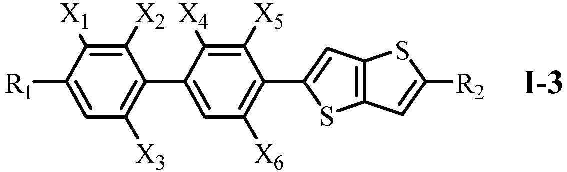 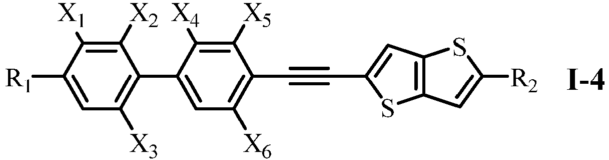 where R₁ and R₂ are each independently selected from the linear alkyl with the number of carbon atoms of 1 to 9 or the linear alkoxy with the number of carbon atoms of 1 to 9.

The compound I-1, the compound I-2, the compound I-3, and the compound I-4 exhibit higher birefringence and higher low-temperature stability. When the compound is used as a liquid crystal monomer for a liquid crystal material, any one, two, three, or four of the compound I-1, the compound I-2, the compound I-3, and the compound I-4 may be used.

Further, the compound used in the liquid crystal material may be one or more of the following compounds. In the following compounds, R₁ and R₂ are each independently selected from the linear alkyl or linear alkoxy with the number of carbon atoms of 1 to 9.

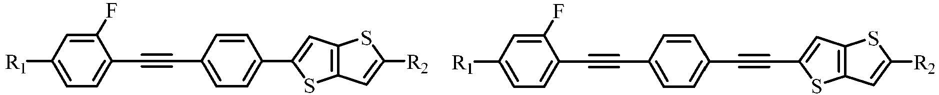

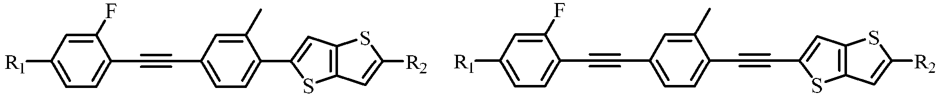

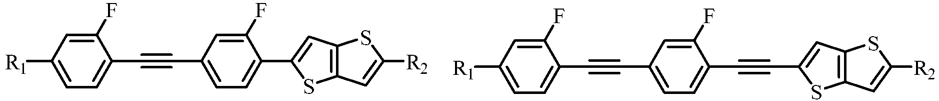

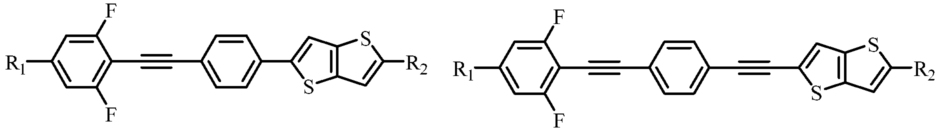

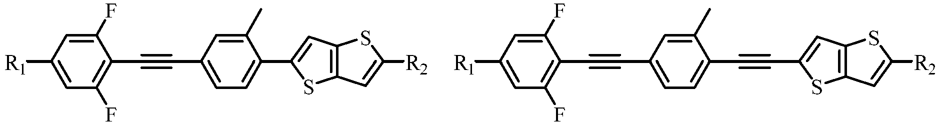

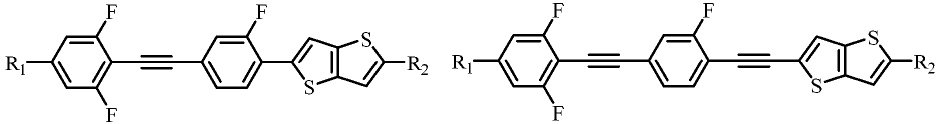

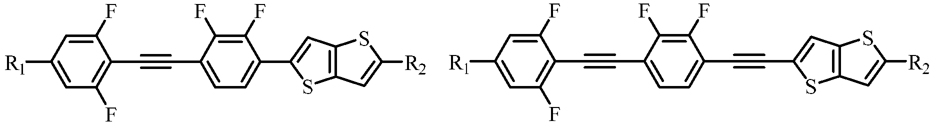

In an example, the compound provided in embodiments of the present disclosure is a liquid crystal monomer compound.

According to another aspect, an embodiment of the present disclosure further provides use of any one of the foregoing compounds in a liquid crystal material.

The compound provided in embodiments of the present disclosure can be used as a liquid crystal monomer, and the compound can be further used with another type of liquid crystal monomer, thereby optimizing the following effects of the liquid crystal material: high birefringence, a wide nematic-phase temperature, excellent low-temperature stability, excellent high-temperature stability, and an excellent ultraviolet resistance capability.

According to still another aspect, an embodiment of the present disclosure further provides a method for preparing any one of the foregoing compounds. The method for preparing the compound includes: determining, based on a chemical structure of the compound, a plurality of monomers for synthesizing the compound; and subjecting the plurality of monomers to a reaction step by step to prepare the compound. A type of the reaction includes but is not limited to at least one of a Sonogashira coupling reaction, a Suzuki coupling reaction, and a halogenation reaction.

In embodiments of the present disclosure, the plurality of monomers for synthesizing the compound are determined based on the chemical structure of the compound. For example, a quantity of monomers for synthesizing the compound is at least 4. First, two monomers are subjected to a reaction to form a first intermediate, and then the first intermediate is subjected to a reaction with another monomer to form a second intermediate, and so on. In this case, the compound is finally prepared by using the plurality of monomers through multi-step reactions.

Both the Sonogashira coupling reaction and the Suzuki coupling reaction include metal catalysts. For the Sonogashira coupling reaction, the Suzuki coupling reaction, and the halogenation reaction, related parameters such as a reaction condition, a solvent condition, and a catalytic environment are adaptively adjusted based on chemical structures of a reaction raw material and a product to be synthesized, to ensure that the reaction can be implemented smoothly. An amount of each related raw material may be calculated based on a molar ratio between raw materials and specific composition of each raw material in a synthetic route.

When the chemical structural formula of the compound is the chemical structural formula I-1, the chemical structural formula I-2, the chemical structural formula I-3, or the chemical structural formula I-4, synthetic routes of the compound of the foregoing types are separately described by using examples.

For the compound I-1 with the chemical structural formula I-1, Z₁ is an alkynyl bond, and Z₂ is a carbon-carbon single bond. A synthetic route corresponding to a method for preparing this compound is shown as follows.

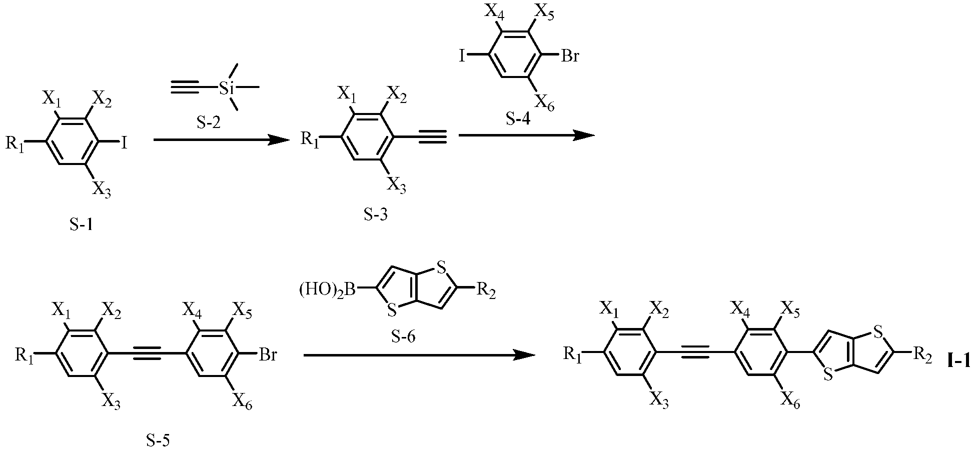

R₁ and R₂ are each independently selected from the linear alkyl with the number of carbon atoms of 1 to 9 or the linear alkoxy with the number of carbon atoms of 1 to 9. X₁ to X₆ are each independently selected from -H, - F, -CH₃, -CH₂CH₃, -OCF₃, cyclopropyl, cyclobutyl, or cyclohexyl.

The method for preparing the compound I-1 is as follows.

A monomer S-1 and a monomer S-2 are subjected to a Sonogashira coupling reaction catalyzed with a metal catalyst, to generate a monomer S-3. For example, a reaction temperature may be 40°C to 120°C, such as 40°C, 50°C, 60°C, 70°C, 80°C, 90°C, 100°C, 110°C, or 120°C. The metal catalyst includes but is not limited to a palladium catalyst.

The monomer S-3 and a monomer S-4 are subjected to a Sonogashira coupling reaction catalyzed with a metal catalyst, to generate a monomer S-5. For example, a reaction temperature may be 40°C to 120°C, such as 40°C, 50°C, 60°C, 70°C, 80°C, 90°C, 100°C, 110°C, or 120°C. The metal catalyst includes but is not limited to a palladium catalyst.

The monomer S-5 and a monomer S-6 are subjected to a Suzuki coupling reaction catalyzed with a metal catalyst, to generate the compound I-1. For example, a reaction temperature may be 60°C to 150°C, such as 60°C, 70°C, 80°C, 90°C, 100°C, 110°C, 120°C, 130°C, 140°C, 150°C, or 160°C. The metal catalyst includes but is not limited to a palladium catalyst.

For the compound I-2 with the chemical structural formula I-2, Z₁ is an alkynyl bond, and Z₂ is an alkynyl bond. A synthetic route corresponding to a method for preparing this compound is shown as follows.

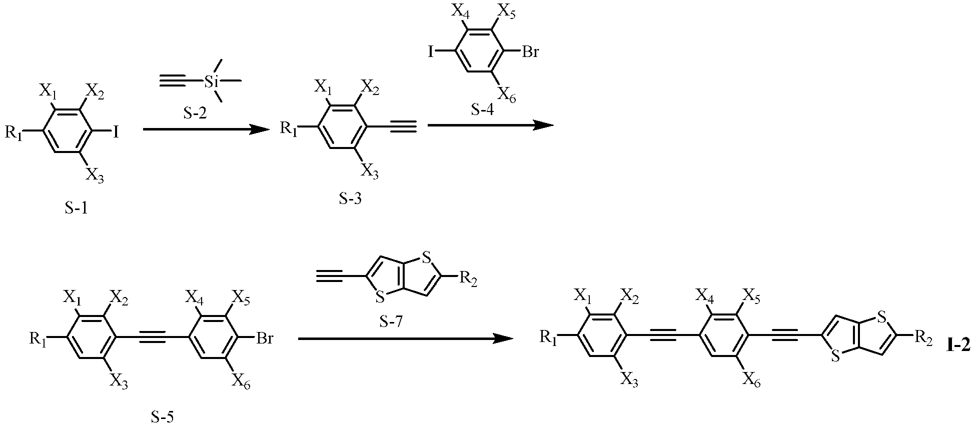

R₁ and R₂ are each independently selected from the linear alkyl with the number of carbon atoms of 1 to 9 or the linear alkoxy with the number of carbon atoms of 1 to 9. X₁ to X₆ are each independently selected from -H, - F, -CH₃, -CH₂CH₃, -OCF₃, cyclopropyl, cyclobutyl, or cyclohexyl.

The method for preparing the compound I-2 is as follows.

A monomer S-1 and a monomer S-2 are subjected to a Sonogashira coupling reaction catalyzed with a metal catalyst, to generate a monomer S-3. For example, a reaction temperature may be 40°C to 120°C, such as 40°C, 50°C, 60°C, 70°C, 80°C, 90°C, 100°C, 110°C, or 120°C. The metal catalyst includes but is not limited to a palladium catalyst.

The monomer S-3 and a monomer S-4 are subjected to a Sonogashira coupling reaction catalyzed with a metal catalyst, to generate a monomer S-5. For example, a reaction temperature may be 40°C to 120°C, such as 40°C, 50°C, 60°C, 70°C, 80°C, 90°C, 100°C, 110°C, or 120°C. The metal catalyst includes but is not limited to a palladium catalyst.

The monomer S-5 and a monomer S-7 are subjected to a Sonogashira coupling reaction catalyzed with a metal catalyst, to generate the compound I-2. For example, a reaction temperature may be 60°C to 150°C, such as 60°C, 70°C, 80°C, 90°C, 100°C, 110°C, 120°C, 130°C, 140°C, 150°C, or 160°C. The metal catalyst includes but is not limited to a palladium catalyst.

For the compound I-3 with the chemical structural formula I-3, Z₁ is a carbon-carbon single bond, and Z₂ is a carbon-carbon single bond. A synthetic route corresponding to a method for preparing this compound is shown as follows.

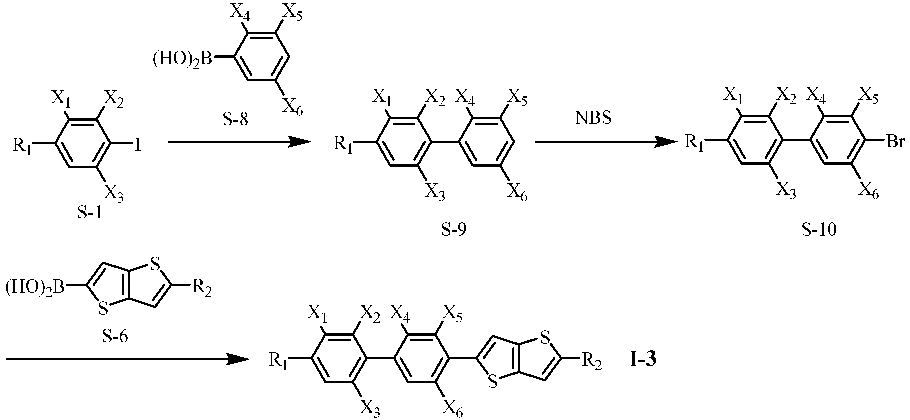

R₁ and R₂ are each independently selected from the linear alkyl with the number of carbon atoms of 1 to 6 or the linear alkoxy with the number of carbon atoms of 1 to 6. X₁ to X₆ are each independently selected from -H, - F, -CH₃, -CH₂CH₃, -OCF₃, cyclopropyl, cyclobutyl, or cyclohexyl.

The method for preparing the compound I-3 is as follows.

A monomer S-1 and a monomer S-8 are subjected to a Suzuki coupling reaction catalyzed with a metal catalyst, to generate a monomer S-9. For example, a reaction temperature may be 60°C to 150°C, such as 60°C, 70°C, 80°C, 90°C, 100°C, 110°C, 120°C, 130°C, 140°C, 150°C, or 160°C. The metal catalyst includes but is not limited to a palladium catalyst.

The monomer S-9 and a monomer N-bromosuccinimide (N-Bromosuccinimide, NBS) are subjected to a bromination reaction, to generate a monomer S-10. For example, a reaction temperature may be -78°C to 60°C, such as -70°C, -60°C, -50°C, -40°C, -30°C, -20°C, -10°C, 0°C, 10°C, 20°C, 30°C, 40°C, 50°C, or 60°C. A catalyst used herein includes but is not limited to a palladium catalyst.

The monomer S-10 and a monomer S-6 are subjected to a Suzuki coupling reaction catalyzed with a metal catalyst, to generate the compound I-3. For example, a reaction temperature may be 60°C to 150°C, such as 60°C, 70°C, 80°C, 90°C, 100°C, 110°C, 120°C, 130°C, 140°C, 150°C, or 160°C. The metal catalyst includes but is not limited to a palladium catalyst.

For the compound I-4 with the chemical structural formula I-4, Z₁ is a carbon-carbon single bond, and Z₂ is an alkynyl bond. A synthetic route corresponding to a method for preparing this compound is shown as follows.

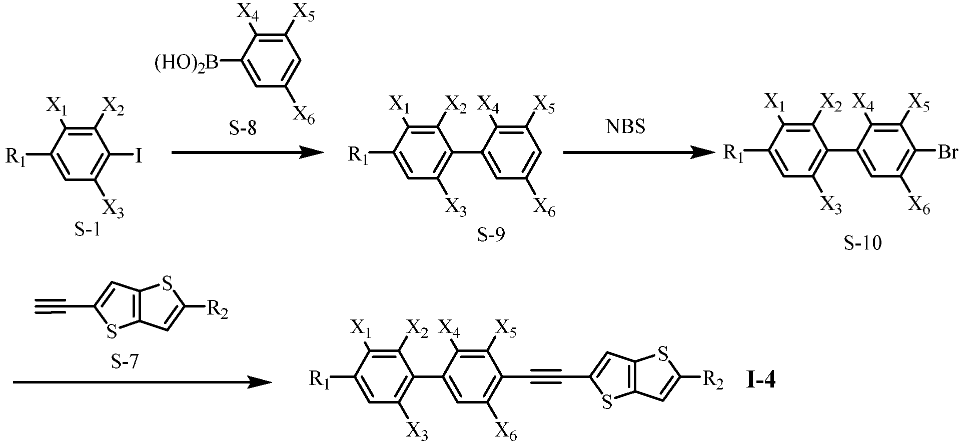

R₁ and R₂ are each independently selected from the linear alkyl with the number of carbon atoms of 1 to 9 or the linear alkoxy with the number of carbon atoms of 1 to 9. X₁ to X₆ are each independently selected from -H, - F, -CH₃, -CH₂CH₃, -OCF₃, cyclopropyl, cyclobutyl, or cyclohexyl.

The method for preparing the compound I-4 is as follows.

A monomer S-1 and a monomer S-8 are subjected to a Suzuki coupling reaction catalyzed with a metal catalyst, to generate a monomer S-9. For example, a reaction temperature may be 60°C to 150°C, such as 60°C, 70°C, 80°C, 90°C, 100°C, 110°C, 120°C, 130°C, 140°C, 150°C, or 160°C. The metal catalyst includes but is not limited to a palladium catalyst.

The monomer S-9 and a monomer N-bromosuccinimide (N-Bromosuccinimide, NBS) are subjected to a bromination reaction, to generate a monomer S-10. For example, a reaction temperature may be -78°C to 60°C, such as -70°C, -60°C, -50°C, -40°C, -30°C, -20°C, -10°C, 0°C, 10°C, 20°C, 30°C, 40°C, 50°C, or 60°C.

The monomer S-10 and a monomer S-7 are subjected to a Sonogashira coupling reaction catalyzed with a metal catalyst, to generate the compound I-4. For example, a reaction temperature may be 40°C to 120°C, such as 40°C, 50°C, 60°C, 70°C, 80°C, 90°C, 100°C, 110°C, or 120°C. The metal catalyst includes but is not limited to a palladium catalyst.

When each of the foregoing compounds is synthesized based on a stepwise polymerization reaction, after each step of the reaction is completed, a reaction product may be purified to achieve refinement. Some applicable purification methods include but are not limited to at least one of chromatography, recrystallization, sublimation, and adsorption.

According to still another aspect, an embodiment of the present disclosure further provides a liquid crystal material. The liquid crystal material includes a first liquid crystal monomer and a second liquid crystal monomer. The first liquid crystal monomer includes any one of the foregoing compounds, and the second liquid crystal monomer is configured to ensure that there is no crystallization phenomenon at a temperature less than or equal to 0°C after the second liquid crystal monomer and the first liquid crystal monomer are mixed.

In some examples, the first liquid crystal monomer is selected from one of the compounds provided in embodiments of the present disclosure. For example, the first liquid crystal monomer includes one of the compound I-1, the compound I-2, the compound I-3, and the compound I-4.

In some other examples, the first liquid crystal monomer is selected from two or more of the compounds provided in embodiments of the present disclosure. For example, the first liquid crystal monomer includes at least two of the compound I-1, the compound I-2, the compound I-3, and the compound I-4.

A mass percentage of the first liquid crystal monomer in the liquid crystal material may be 0.5% to 70%. For example, the mass percentage of the first liquid crystal monomer in the liquid crystal material includes but is not limited to 1% to 50%, 1% to 30%, and 3% to 20%. Some examples of the mass percentage of the first liquid crystal monomer include but are not limited to 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, and 70%.

By using any one of the foregoing compounds provided in embodiments of the present disclosure as the first liquid crystal monomer, based on the synergistic effect of the first liquid crystal monomer and the second liquid crystal monomer, the liquid crystal material provided in embodiments of the present disclosure exhibits at least high birefringence, a wide nematic-phase temperature, and excellent low-temperature stability.

In an embodiment of the present disclosure, the second liquid crystal monomer is configured to ensure that there is no crystallization phenomenon at a temperature less than or equal to 0°C after the second liquid crystal monomer and the first liquid crystal monomer are mixed.

For example, the temperature less than or equal to 0°C may be a temperature of 0°C, a temperature of - 5°C, a temperature of -10°C, a temperature of -15°C, a temperature of -20°C, or the like.

The first liquid crystal monomer and the second liquid crystal monomer are combined. The synergistic effect of the first liquid crystal monomer and the second liquid crystal monomer allows the prepared liquid crystal material to have advantages such as high birefringence, a wide nematic-phase temperature, excellent low-temperature stability, excellent high-temperature stability, and an excellent ultraviolet resistance capability.

For example, some second liquid crystal monomers that have good compatibility with the first liquid crystal monomer and that can optimize performance of the liquid crystal material based on the synergistic effect with the first liquid crystal monomer are selected from at least one of a compound II-1, a compound II-2, a compound II-3, a compound II-4, a compound II-5, a compound II-6, a compound II-7, and a compound II-8.

A chemical structural formula II-1 corresponding to the compound II-1, a chemical structural formula II-2 corresponding to the compound II-2, a chemical structural formula II-3 corresponding to the compound II-3, a chemical structural formula II-4 corresponding to the compound II-4, a chemical structural formula II-5 corresponding to the compound II-5, a chemical structural formula II-6 corresponding to the compound II-6, a chemical structural formula II-7 corresponding to the compound II-7, and a chemical structural formula II-8 corresponding to the compound II-8 are separately shown as follows: 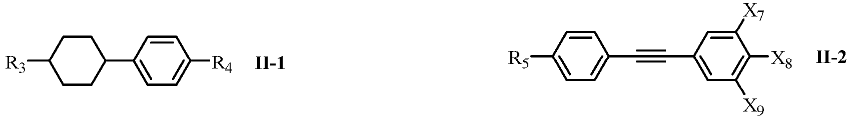 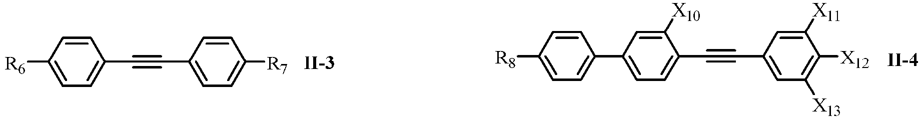 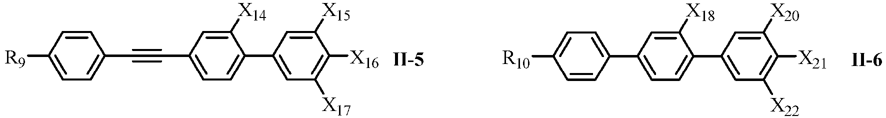 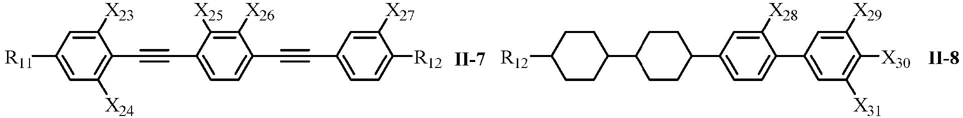 where R₃ to R₁₂ are each independently selected from one of linear alkyl with a number of carbon atoms of 1 to 9 and linear alkoxy with a number of carbon atoms of 1 to 9; and X₇ to X₃₁ are each independently selected from -H, -F, -CH₃, -CH₂CH₃, or -OCF₃.

For the compound II-2, some specific examples include but are not limited to:

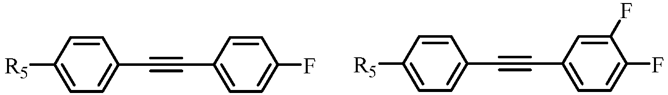

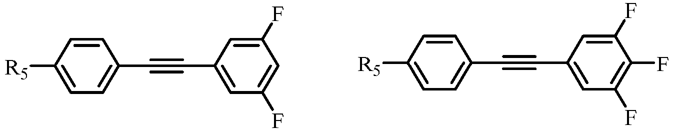

For the compound II-4, some specific examples include but are not limited to:

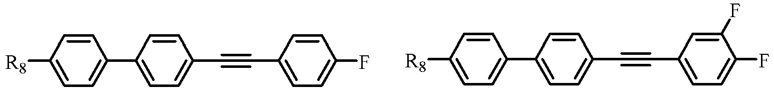

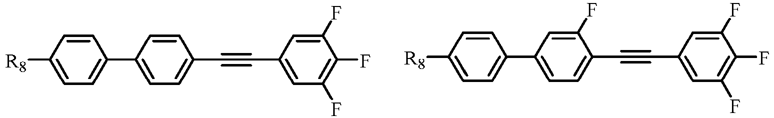

For the compound II-5, some specific examples include but are not limited to:

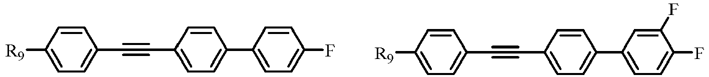

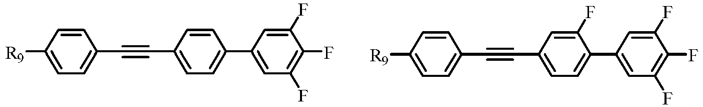

For the compound II-6, some specific examples include but are not limited to:

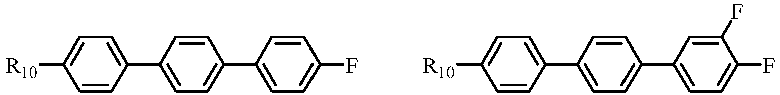

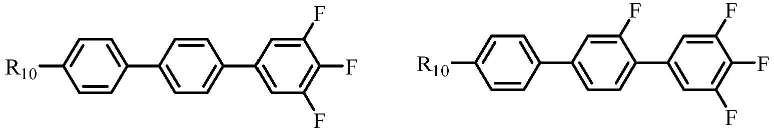

For the compound II-7, some specific examples include but are not limited to:

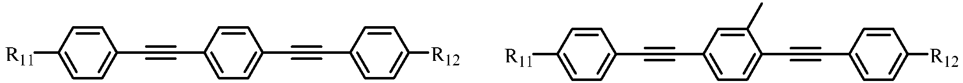

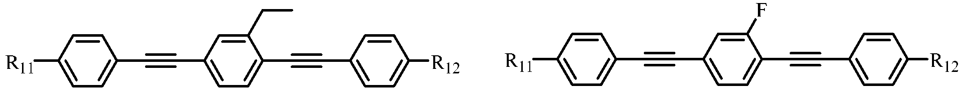

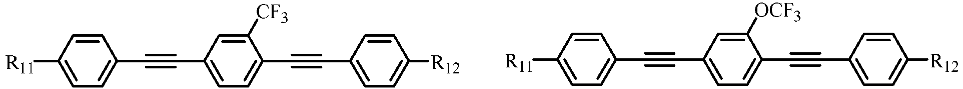

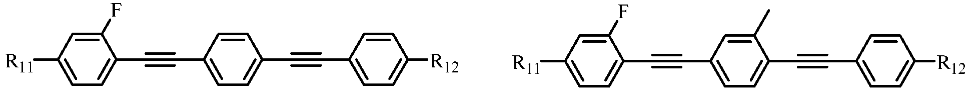

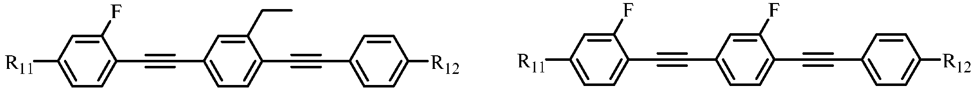

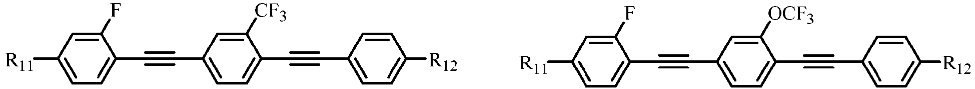

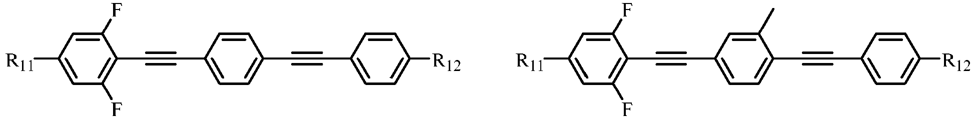

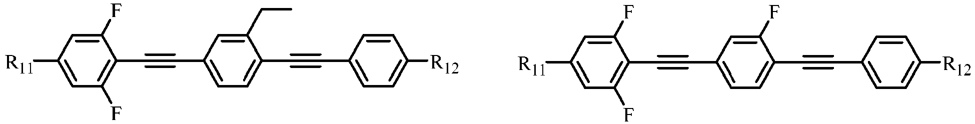

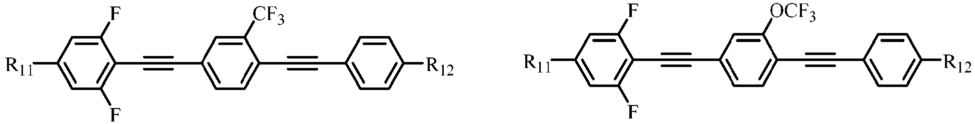

For the compound II-8, some specific examples include but are not limited to:

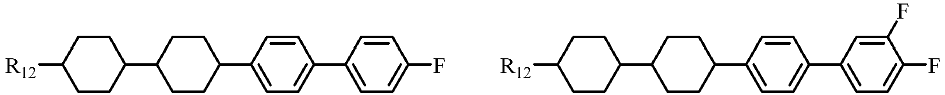

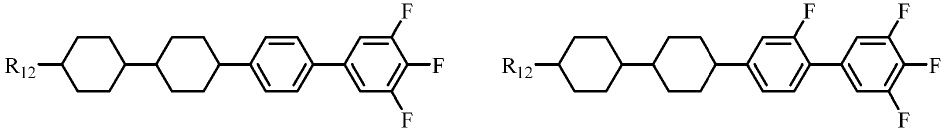

The foregoing second liquid crystal monomers are all liquid crystal compounds known in the art. These second liquid crystal monomers have good compatibility with the first liquid crystal monomer. In particular, at a low temperature below -20°C, the second liquid crystal monomer and the first liquid crystal monomer can be well miscible without crystallization. This is particularly advantageous for improving low-temperature stability of the liquid crystal material. In addition, synergistic formulating of the second liquid crystal monomer and the first liquid crystal monomer can further improve birefringence, high-temperature stability, and an ultraviolet resistance capability of the liquid crystal material, and expand a nematic-phase temperature range of the liquid crystal material.

The second liquid crystal monomer is selected from at least one of the compound II-1, the compound II-2, the compound II-3, the compound II-4, the compound II-5, the compound II-6, the compound II-7, and the compound II-8.

For example, a quantity of liquid crystal compounds included in the second liquid crystal monomer may be 1, 2, 3, 4, 5, 6, 7, 8, 9, 10, 11, 12, 13, 14, 15, 16, 17, 18, 19, or 20.

The compound provided in embodiments of the present disclosure is defined as a compound I. The compound II-1, the compound II-2, and the compound II-3 are selected to be blended with the compound I, helping improve low-temperature stability of a composition. The compound II-4, the compound II-5, and the compound II-6 are selected to be blended with the compound I, helping increase birefringence and a dielectric constant of a composition. The compound II-8 is selected to be blended with the compound I, helping expand a nematic-phase temperature range of a composition and suppress formation of a smectic phase.

In some examples, a sum of a mass percentage of the first liquid crystal monomer and a mass percentage of the second liquid crystal monomer is 100%. For example, the mass percentage of the second liquid crystal monomer may be 0.5% to 95%, including 1% to 70%, 5% to 60%, 10% to 50%, and the like.

Some examples of the mass percentage of the second liquid crystal monomer include but are not limited to: 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 86%, 87%, 88%, 89%, 90%, 91%, 92%, 93%, 94%, and 95%.

In some other examples, the liquid crystal material provided in embodiments of the present disclosure further includes an additive. In other words, the liquid crystal material includes the first liquid crystal monomer, the second liquid crystal monomer, and an additive. The additive is selected from at least one of an ultraviolet absorber, an ultraviolet stabilizer, and an antioxidant.

Based on a total mass of the first liquid crystal monomer and the second liquid crystal monomer being 100 parts, a mass of the additive is 0.01 part to 10 parts. The mass of the additive in the liquid crystal material is 0.05 part, 0.1 part, 0.15 part, 0.2 part, 0.3 part, 0.4 part, 0.5 part, 0.6 part, 0.7 part, 0.8 part, 0.9 part, 1 part, 2 parts, 3 parts, 4 parts, 5 parts, 6 parts, 7 parts, 8 parts, 9 parts, 10 parts, or the like.

One or more of the foregoing additives are added to the liquid crystal material, so that an ultraviolet resistance capability and high-temperature stability of the liquid crystal material can be further improved.

For example, some suitable ultraviolet absorbers are selected from at least one of a benzotriazole ultraviolet absorber and a triazine ultraviolet absorber; the ultraviolet stabilizer is selected from a hindered amine ultraviolet stabilizer; and the antioxidant is selected from a hindered phenol antioxidant.

For example, chemical structural formulas of some suitable additives are shown as follows. In addition, in the following chemical structural formulas, n represents an integer from 1 to 20, and R₁₃ to R₁₆ are each selected from linear alkyl with a number of carbon atoms of 1 to 10 or linear alkoxy with a number of carbon atoms of 1 to 10.

Some examples of the hindered amine ultraviolet stabilizer are shown as follows:

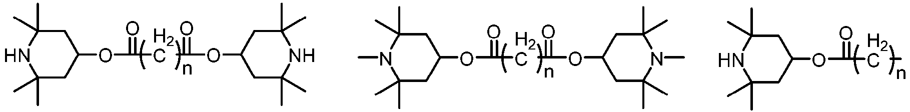

Some examples of the benzotriazole ultraviolet absorber are shown as follows:

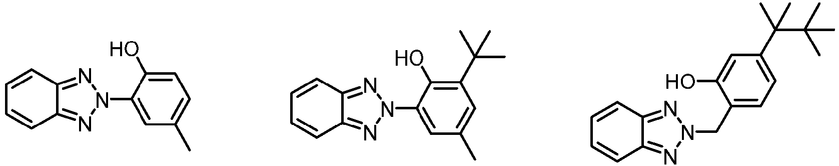

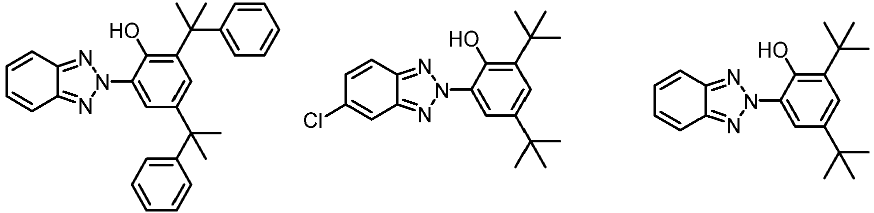

Some examples of the triazine ultraviolet absorber are shown as follows:

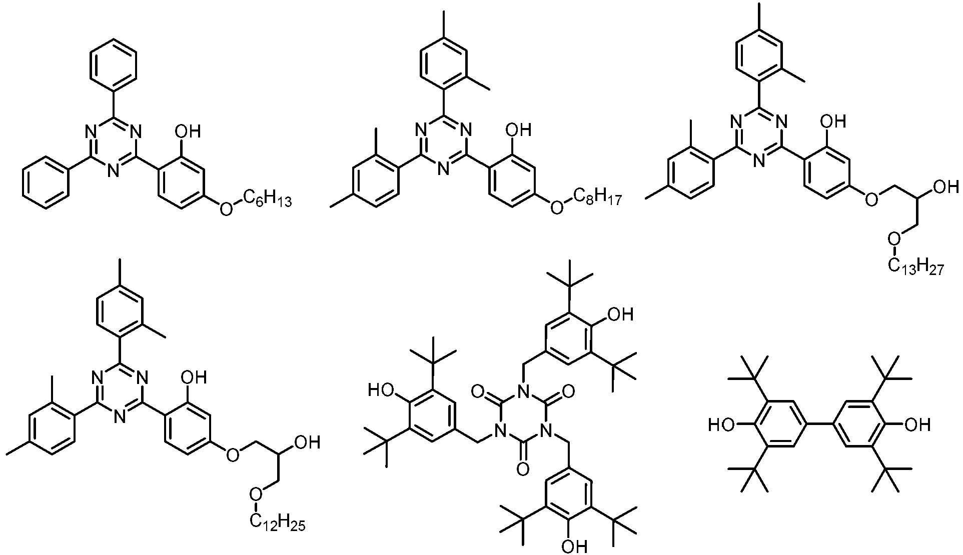

Some examples of the hindered phenol antioxidant (including a fully hindered phenol antioxidant and a partially hindered phenol antioxidant) are shown as follows:

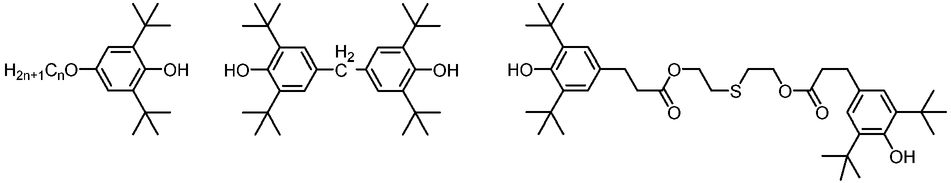

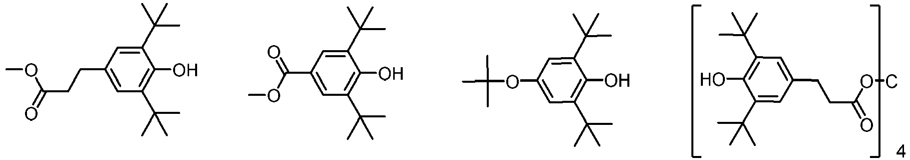

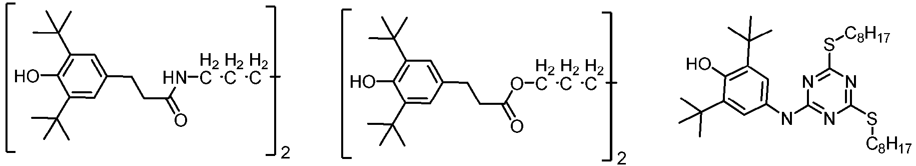

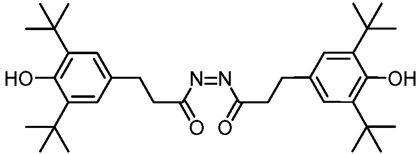

The liquid crystal material provided in embodiments of the present disclosure is a composition (that is, a liquid crystal composition). When the liquid crystal material provided in embodiments of the present disclosure is prepared, components may be mixed in proportions of the components by using one or more of the following mixing methods: a method of dissolving a mixture by heating, an ultrasonication method, a suspension mixing method, and the like, to prepare the liquid crystal material with uniform texture.

The liquid crystal material in embodiments of the present disclosure has at least one of the following characteristics: no crystallization at -60°C to 0°C; no color change at 0°C to 150°C; and no color change at a UV intensity less than or equal to 80 mW/cm². In some examples, the liquid crystal material in embodiments of the present disclosure has all the foregoing three characteristics.

The characteristic "no crystallization at -60°C to 0°C" indicates that the liquid crystal material exhibits excellent low-temperature stability, so that the liquid crystal material can be stably stored at low temperatures. For example, a storage temperature at which the liquid crystal material does not undergo crystallization includes but is not limited to -60°C, -55°C, -50°C, -45°C, -40°C, -35°C, -30°C, -25°C, -20°C, -15°C, -10°C, -5°C, and 0°C.

The characteristic "no color change at 0°C to 150°C" indicates that the liquid crystal material exhibits excellent high-temperature stability, so that the liquid crystal material can be stably effective at high temperatures. For example, an operating temperature at which the liquid crystal material does not undergo color change includes but is not limited to 30°C, 40°C, 50°C, 60°C, 70°C, 80°C, 90°C, 100°C, 110°C, 120°C, 130°C, and 140°C.

The characteristic "no color change at a UV intensity less than or equal to 80 mW/cm²" indicates that the liquid crystal material has an excellent UV resistance characteristic, so that the liquid crystal material can be stably effective under UV conditions. For example, the UV intensity at which the liquid crystal material does not undergo color change includes but is not limited to 10 mW/cm², 20 mW/cm², 30 mW/cm², 40 mW/cm², 50 mW/cm², 60 mW/cm², 70 mW/cm², and 80 mW/cm².

According to still another aspect, an embodiment of the present disclosure further provides an optoelectronic device. The optoelectronic device includes an encapsulation structure and a liquid crystal material encapsulated in the encapsulation structure, where the liquid crystal material is the foregoing liquid crystal material according to embodiments of the present disclosure.

The optoelectronic device provided in embodiments of the present disclosure has all advantages of the foregoing liquid crystal material.

For example, the optoelectronic device includes but is not limited to a wavelength selective switch, a microwave antenna, or a display device. The display device includes but is not limited to a display (such as a liquid crystal display, an on-board head-up display, or a holographic display), a liquid crystal lamp (for example, an on-board smart liquid crystal car lamp), and glasses (for example, AR/VR glasses).

In some examples, an embodiment of the present disclosure further provides a wavelength selective switch including a liquid crystal layer. The liquid crystal layer is formed by using any one of the foregoing liquid crystal materials in embodiments of the present disclosure.

The following describes specific implementations of embodiments of the present disclosure in more detail. Although the following describes specific implementations of embodiments of the present disclosure, it should be understood that embodiments of the present disclosure may be implemented in various forms and should not be limited to the implementations described herein. If no specific technique or condition is specified in embodiments, a technique or condition described in the literature in the art or a product specification shall be followed. Reagents or instruments used with no indication of manufacturers are conventional products that are commercially available.

The following defines or formulates an evaluation criterion for related parameters in embodiments, specifically as follows.
(1) Clearing points (Cp) of a liquid crystal monomer and a liquid crystal composition are obtained through testing using a differential scanning calorimeter (Differential scanning calorimetry, DSC). The testing for the clearing point is performed at a heating rate of 5°C/min.
(2) Birefringence of a liquid crystal monomer and birefringence of a liquid crystal composition are measured by using an Abbe refractometer at 25°C and at a wavelength of 589 nm.
(3) Δε represents dielectric anisotropy of a liquid crystal monomer/liquid crystal composition at 25°C and at 1 kHz. ¹H NMR represents a hydrogen nuclear magnetic resonance spectrum, ¹³C NMR represents a carbon nuclear magnetic resonance spectrum, and ¹⁹F NMR represents a fluorine nuclear magnetic resonance spectrum. A test instrument is a 400 MHz or 500 MHz nuclear magnetic resonance spectrometer from Bruker.
(4) Low-temperature stability test: A liquid crystal composition sample is stored in a refrigerator at - 30°C for 20 days. No crystallization indicates excellent low-temperature stability, while crystallization indicates poor low-temperature stability that requires further improvement.
(5) UV resistance capability test: A liquid crystal composition is injected into a liquid crystal cell through capillary filling. After exposure to a 365 nm ultraviolet lamp at an irradiation intensity of 50 mW/cm² to 10 J, the liquid crystal cell is evaluated by a color change degree, and the color change degree is used as an evaluation indicator for an ultraviolet resistance capability. An evaluation criterion is shown in Table 1.

**Table 1**

| | | | |
|---|---|---|---|
| UV resistance capability | Excellent | Good | Poor |
| Color change | Colorless and transparent | Slightly yellow | Yellow |

(6) High-temperature stability test: A liquid crystal composition is injected into a liquid crystal cell through capillary filling. The liquid crystal cell is irradiated at 365 nm to cure a sealing adhesive, and heated in an oven at a high temperature of 100°C for 100 hours. Then, the liquid crystal cell is evaluated by a color change degree, and the color change degree is used as an evaluation indicator for high-temperature stability. An evaluation criterion is shown in Table 2:

**Table 2**

| | | | |
|---|---|---|---|
| High-temperature stability | Excellent | Good | Poor |
| Color change | Colorless and transparent | Slightly yellow | Yellow |

In addition, for compounds corresponding to liquid crystal monomers and additives in the following embodiments, each of these compounds includes a plurality of groups or bonds, and related groups or bonds are represented by codes shown in Table 3. In this way, based on positions of a plurality of groups and bonds in a chemical structure of a corresponding compound, codes are combined in a sequence to clearly name the compound. In addition, the chemical structure of the compound can be deduced based on the codes of the compound.

**Table 3**

| Group/Bond | Code | Group name |
|---|---|---|
| 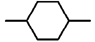 | C | 1,4-cyclohexylene |
| 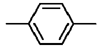 | P | 1,4-phenylene |
| 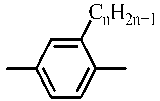 | P(n) (n is a positive integer from 1 to 10) | 2-alkyl-1,4-phenylene |
| 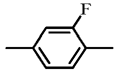 | G | 2-fluoro-1,4-phenylene |
| 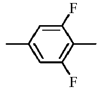 | U | 2,5-difluoro-1,4-phenylene |
| 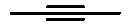 | T | Alkynyl |
| 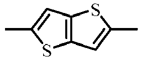 | S | Thieno[3,2-b]thiophene |
| 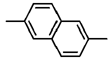 | M | 1,6-naphthalenediyl |
| 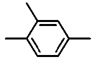 | V | 3-methyl-1,4-phenylene |
| -CₙH₂ₙ₊₁ or -CₘH₂ₘ₊₁ | n or m, n and m are positive integers from 1 to 10 | Alkyl |
| -OₙCₙH₂ₙ₊₁ or -OCₘH₂ₘ₊₁ | On or Om, n and m are positive integers from 1 to 10 | Alkoxy |
| -F | F | Fluorine |
| -NCS | NCS | Isothiocyanate |
| 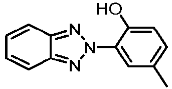 | UV-P | 2-(2'-hydroxy-5'-methylphenyl)benzotriazole |
| 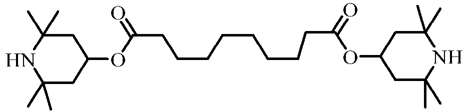 | UV-770 | Bis(2,2,6,6-tetramethyl-4-piperidyl)sebacate |

### Embodiment 1

Embodiment 1 provides a compound named 3UTP(1)S4 with a chemical structural formula shown as follows:

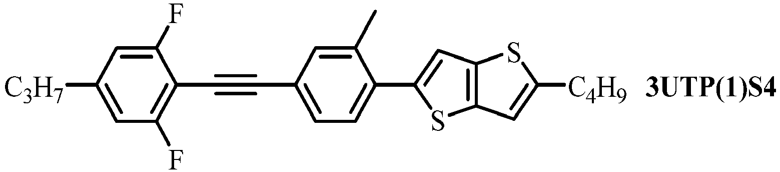

A synthetic route of the compound 3UTP(1)S4 is shown as follows:

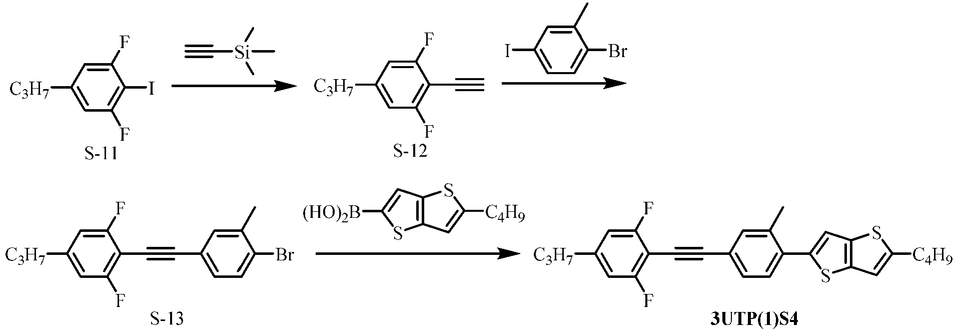

With reference to the synthetic route of the compound 3UTP(1)S4, a method for preparing the compound 3UTP(1)S4 is shown as follows.

### (1) Preparation of 2-ethynyl-1,3-difluoro-5-propylbenzene (monomer S-12)

Under nitrogen protection, 1,3-difluoro-2-iodo-5-propylbenzene (monomer S-11), ethynyltrimethylsilane, copper iodide, bis(triphenylphosphine)palladium(II) chloride, and a triethylamine solution are added to a round-bottom flask, and heated and stirred to react. After the reaction system is cooled down to room temperature, an extractant is added for extraction (where the extractant includes but is not limited to ethyl acetate). Organic phases are combined, dried over magnesium sulfate, and spin-dried under reduced pressure. Potassium carbonate and a mixture of methanol and dichloromethane (in any volume ratio) are added to the system, and further stirred. Then, distilled water is added, and an extractant is added for extraction. Organic phases are combined, spin-dried under reduced pressure, and separated by column chromatography, to obtain the monomer S-12.

### (2) Preparation of 2-((4-bromo-3-methylphenyl)ethynyl)-1,3-difluoro-5-propylbenzene (monomer S-13)

Under nitrogen protection, 2-ethynyl-1,3-difluoro-5-propylbenzene (monomer S-12), 2-bromo-5-iodotoluene, copper iodide, bis(triphenylphosphine)palladium(II) chloride, and a triethylamine solution are added to a round-bottom flask, and heated and stirred to react. After the system is cooled down to room temperature, an extractant is added for extraction. Organic phases are combined, dried over magnesium sulfate, spin-dried under reduced pressure, and separated by column chromatography, to obtain the monomer S-13.

### (3) Preparation of the compound 3UTP(1)S4

Under nitrogen protection, 2-((4-bromo-3-methylphenyl)ethynyl)-1,3-difluoro-5-propylbenzene (monomer S-13), 5-butyl-2-thieno[3,2-b]thiopheneboronic acid, potassium carbonate, tetrakis(triphenylphosphine)palladium, and a mixture of 1,4-dioxane and water (provided that a volume ratio of 1,4-dioxane to water is greater than 1) are added to a 500 mL round-bottom flask, and heated and stirred to react. The reaction system is cooled down to room temperature. An extractant is added for extraction. Organic phases are combined, dried over magnesium sulfate, spin-dried under reduced pressure, and separated by column chromatography, to obtain the compound 3UTP(1)S4.

The compound 3UTP(1)S4 is subjected to DSC testing. Through testing, phase transition temperatures of the compound 3UTP(1)S4 are Cr 73.9°C and N 155.4°CI (where Cr represents a crystallization point, N represents a nematic phase, and I represents a clearing point; and the data indicates that a crystallization temperature is 73.9°C, the nematic phase is present until 155.4°C, and the compound is clear above 155.4°C), and birefringence △n (589 nm, 25°C) of the compound 3UTP(1)S4 is 0.365.

Structural characterization of the compound 3UTP(1)S4 is performed by hydrogen nuclear magnetic resonance spectroscopy, carbon nuclear magnetic resonance spectroscopy, and fluorine nuclear magnetic resonance spectroscopy. Testing results are as follows:
¹H-NMR (500 M, CDCl₃) δ(ppm): 7.51 (s, 1H), 7.44 (s, 2H), 7.20 (s, 1H), 6.98 (s, 1H), 6.79-6.78 (d, 2H), 2.94-2.91 (t, 2H), 2.62-2.59 (t, 2H), 2.50 (s, 3H), 1.77-1.71 (m, 2H), 1.69-1.64 (m, 2H), 1.48-1.44 (m, 2H), 1.00-0.96 (m, 6H).

¹³C-NMR (125 M, CDCl₃) δ(ppm): 163.79, 163.74, 161.78, 161.73, 148.28, 146.20, 146.13, 146.06, 142.38, 138.90, 137.47, 136.12, 135.08, 134.02, 130.32, 129.25, 122.11, 119.13, 116.26, 111.29, 111.25, 111.13, 111.10, 99.73, 99.58, 99.42, 98.26, 98.23, 37.88, 33.71, 30.85, 23,77, 22.17, 21.14, 13.79, 13.56.

¹⁹F NMR (376 M, CDCl₃) δ(ppm): -108.54, -108,56.

It can be learned that the chemical structure of the compound 3UTP(1)S4 prepared in Embodiment 1 is consistent with the chemical structural formula thereof.

### Embodiment 2

Embodiment 2 provides a compound named 3UTGTS5 with a chemical structural formula shown as follows:

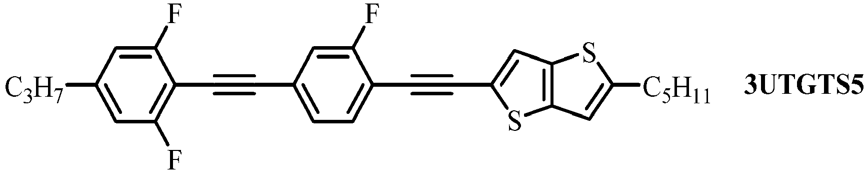

A synthetic route of the compound 3UTGTS5 is shown as follows:

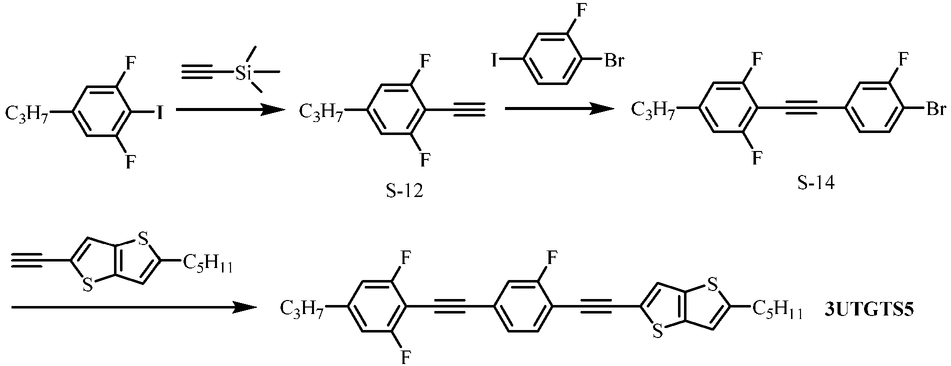

With reference to the synthetic route of the compound 3UTGTS5, a method for preparing the compound 3UTGTS5 is shown as follows.

### (1) Preparation of 2-ethynyl-1,3-difluoro-5-propylbenzene (monomer S-12)

A method for preparing 2-ethynyl-1,3-difluoro-5-propylbenzene (monomer S-12) is the same as that in Embodiment 1. Details are not described herein again.

### (2) Preparation of 2-((4-bromo-3-fluorophenyl)ethynyl)-1,3-difluoro-5-propylbenzene (monomer S-14)

Under nitrogen protection, 2-ethynyl-1,3-difluoro-5-propylbenzene (monomer S-12), 1-bromo-2-fluoro-4-iodobenzene, copper iodide, bis(triphenylphosphine)palladium(II) chloride, and a triethylamine solution are added to a round-bottom flask, and heated and stirred to react. After the system is cooled down to room temperature, an extractant is added for extraction. Organic phases are combined, dried over magnesium sulfate, spin-dried under reduced pressure, and separated by column chromatography, to obtain the monomer S-14.

### (3) Preparation of the compound 3UTGTS5

Under nitrogen protection, 2-((4-bromo-3-fluorophenyl)ethynyl)-1,3-difluoro-5-propylbenzene (monomer S-14), 2-ethynyl-5-pentylthieno[3,2-b]thiophene, copper iodide, tetrakis(triphenylphosphine)palladium, and a mixture of N,N-dimethylformamide and triethylamine (provided that a volume ratio of N,N-dimethylformamide to triethylamine is greater than 1) are added to a round-bottom flask, and heated and stirred to react. After the system is cooled down to room temperature, an extractant is added for extraction. Organic phases are combined, dried over magnesium sulfate, spin-dried under reduced pressure, and separated by column chromatography, to obtain the compound 3UTGTS5.

The compound 3UTGTS5 is subjected to DSC testing. Through testing, a phase transition temperature of the compound 3UTGTS5 is Cr 94.1°CI, and birefringence △n (589 nm, 25°C) of the compound 3UTGTS5 is 0.525.

Structural characterization of the compound 3UTGTS5 is performed by hydrogen nuclear magnetic resonance spectroscopy, carbon nuclear magnetic resonance spectroscopy, and fluorine nuclear magnetic resonance spectroscopy. Testing results are as follows:
¹H-NMR (500 M, CDCl₃) δ(ppm): 7.49-7.46 (t, 1H), 7.42 (s, 1H), 7.34-7.29 (m, 2H), 6.92 (s, 1H), 6.79-6.77 (d, 2H), 2.91-2.88 (t, 2H), 2.61-2.58 (t, 2H), 1.77-1.71 (m, 2H), 1.69-1.62 (m, 2H), 1.39-1.37 (m, 4H), 0.97-0.91 (m, 6H).

¹³C-NMR (125 M, CDCl₃) δ(ppm): 163.80, 163.75, 162.83, 161.78, 160.82, 151.10, 146.88, 146.81, 140.48, 135.47, 132.88, 127.48, 124.41, 124.34, 121.92, 119.05, 118.40, 116.32, 113.20, 112.30, 111.36, 111.32, 111.20, 111.17, 99.13, 98.97, 98.81, 96.12, 89.42, 85.44, 79.42, 37.91, 31.27, 31.24, 31.09, 23.71, 22.37, 14.42, 13.53.

¹⁹F NMR (376 M, CDCl₃) δ(ppm): -108.19, -108.21, -109.59, -109.61.

It can be learned that the chemical structure of the compound 3UTGTS5 prepared in Embodiment 2 is consistent with the chemical structural formula thereof.

### Embodiment 3

Embodiment 3 provides a compound named 4UTP(1)S3 with a chemical structural formula shown as follows:

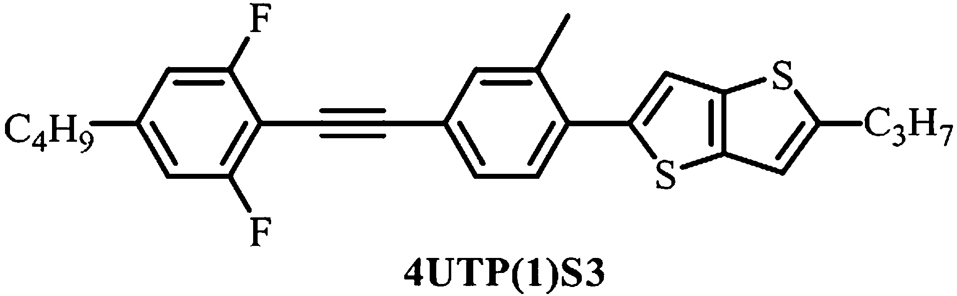

A synthetic route of the compound 4UTP(1)S3 is shown as follows:

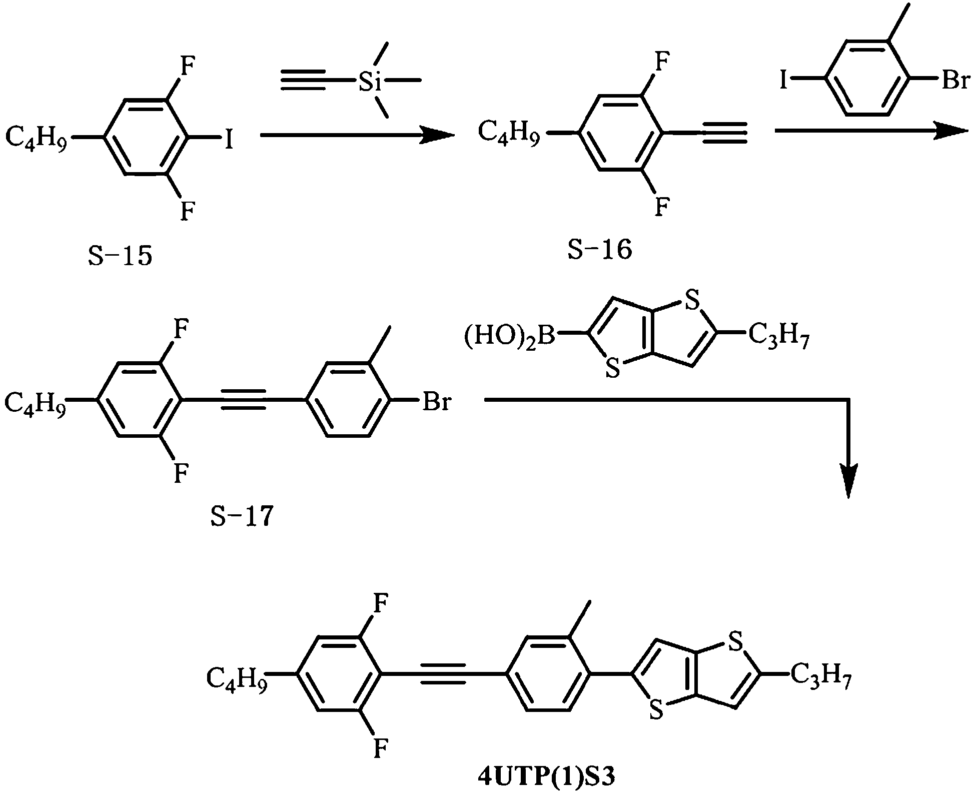

With reference to the synthetic route of the compound 4UTP(1)S3, a method for preparing the compound 4UTP(1)S3 is shown as follows.

### (1) Preparation of 2-ethynyl-1,3-difluoro-5-butylbenzene (monomer S-16)

Under nitrogen protection, 1,3-difluoro-2-iodo-5-butylbenzene (monomer S-15), ethynyltrimethylsilane, copper iodide, bis(triphenylphosphine)palladium(II) chloride, and a triethylamine solution are added to a round-bottom flask, and heated and stirred to react. After the reaction system is cooled down to room temperature, an extractant is added for extraction. Organic phases are combined, dried over magnesium sulfate, and spin-dried under reduced pressure. Potassium carbonate and a mixture of methanol and a dichloromethane solution (in any volume ratio) are added to the system, and further stirred to react. Then, distilled water is added, and an extractant is added for extraction. Organic phases are combined, spin-dried under reduced pressure, and separated by column chromatography, to obtain the monomer S-16.

### (2) Preparation of 2-((4-bromo-3-methylphenyl)ethynyl)-1,3-difluoro-5-butylbenzene (monomer S-17)

Under nitrogen protection, 2-ethynyl-1,3-difluoro-5-butylbenzene (monomer S-16), 2-bromo-5-iodotoluene, copper iodide, bis(triphenylphosphine)palladium(II) chloride, and a triethylamine solution are added to a round-bottom flask, and heated and stirred to react. After the system is cooled down to room temperature, an extractant is added for extraction. Organic phases are combined, dried over magnesium sulfate, spin-dried under reduced pressure, and separated by column chromatography, to obtain the monomer S-17.

### (3) Preparation of the compound 4UTP(1)S3

Under nitrogen protection, 2-((4-bromo-3-methylphenyl)ethynyl)-1,3-difluoro-5-butylbenzene (monomer S-17), 5-propyl-2-thieno[3,2-b]thiopheneboronic acid, potassium carbonate, tetrakis(triphenylphosphine)palladium, and a mixture of solvent 1,4-dioxane and water (provided that a volume ratio of 1,4-dioxane to water is greater than 1) are added to a round-bottom flask, and heated and stirred to react. The reaction system is cooled down to room temperature. An extractant is added for extraction. Organic phases are combined, dried over magnesium sulfate, spin-dried under reduced pressure, and separated by column chromatography, to obtain the compound 4UTP(1)S3.

The compound 4UTP(1)S3 is subjected to DSC testing. Through testing, phase transition temperatures of the compound 4UTP(1)S3 are Cr 92.0°C and N 160.2°CI, and birefringence △n (589 nm, 25°C) of the compound 4UTP(1)S3 is 0.363.

Structural characterization of the compound 4UTP(1)S3 is performed by hydrogen nuclear magnetic resonance spectroscopy, carbon nuclear magnetic resonance spectroscopy, and fluorine nuclear magnetic resonance spectroscopy. Testing results are as follows:
¹H-NMR (500 M, CDCl₃) δ(ppm): 7.50 (s, 1H), 7.44 (s, 2H), 7.20 (s, 1H), 6.98 (s, 1H), 6.79-6.77 (d, 2H), 2.91-2.88 (t, 2H), 2.64-2.61 (t, 2H), 2.50 (s, 3H), 1.80-1.76 (m, 2H), 1.64-1.58 (m, 2H), 1.41-1.35 (m, 2H), 1.05-1.02 (t, 3H), 0.97-0.94 (t, 3H).

¹³C-NMR (125 M, CDCl₃) δ(ppm): 163.78, 163.73, 161.77, 161.72, 148.03, 146.44, 146.36, 146.29, 142.39, 138.89, 137.50, 136.55, 135.07, 134.01, 131.04, 129.24, 122.71, 119.12, 116.35, 111.23, 111.20, 111.08, 111.04, 99.67, 99.51, 98.17, 36.04, 33.20, 32.72, 25.84, 22.98, 21.13, 13.79, 13.64.

¹⁹F NMR (376 M, CDCl₃) δ(ppm): -108.54, -108,56.

It can be learned that the chemical structure of the compound 4UTP(1)S3 prepared in Embodiment 3 is consistent with the chemical structural formula thereof.

### Embodiment 4

Embodiment 4 provides a liquid crystal composition. The liquid crystal composition includes a first liquid crystal monomer, a second liquid crystal monomer, and an additive. The first liquid crystal monomer is the compound 4UTP(1)S3 prepared in Embodiment 3. The second liquid crystal monomer includes a plurality of liquid crystal compounds, and chemical structures of these liquid crystal compounds can be deduced based on names of corresponding codes in Table 4.

A sum of a mass percentage of the first liquid crystal monomer and mass percentages of all the liquid crystal compounds in the second liquid crystal monomer is 100%. A total mass of the first liquid crystal monomer and the second liquid crystal monomer is defined as 100 parts by weight. Then, a mass of the additive is 0.6 part by weight.

The formulation of the liquid crystal composition is shown in Table 4. The components in Table 4 are added to a sample vial with a magnetic stir bar, heated and stirred until clear, and cooled down to room temperature, to prepare the liquid crystal composition.

**Table 4**

| Component | Parts by mass |
|---|---|
| 4UTP(1)S3 | 6.0 |
| 3CPO2 | 3.0 |
| 2PTGF | 3.0 |
| 4UTPP3 | 6.0 |
| 3UTPP4 | 6.0 |
| 4PTGUF | 10.0 |
| 3PTGUF | 5.0 |
| 5PTGUF | 5.0 |
| 2PTGGF | 6.0 |
| 3PTGGF | 4.0 |
| 4PTGGF | 8.0 |
| 5PTGGF | 5.0 |
| 3CCPUF | 3.0 |
| 2UTGTP5 | 6.0 |
| 3UTGP5 | 6.0 |
| 3UTP(1)TP2 | 6.0 |
| 4UTP(1)TP3 | 6.0 |
| 4UTP(1)TP2 | 6.0 |
| UV-P | 0.4 |
| UV-770 | 0.2 |

The liquid crystal composition provided in Embodiment 4 exhibits good mutual miscibility among the components. Through testing, the liquid crystal composition provided in Embodiment 4 has a clearing point (Cp) of 124°C and birefringence △n of 0.3279 at 25°C and at 589 nm.

### Embodiment 5

Embodiment 5 provides a liquid crystal composition. The liquid crystal composition includes a first liquid crystal monomer, a second liquid crystal monomer, and an additive. The first liquid crystal monomer is the compound 3UTGTS5 prepared in Embodiment 2. The second liquid crystal monomer includes a plurality of liquid crystal compounds, and chemical structures of these liquid crystal compounds can be deduced based on names of corresponding codes in Table 5.

A sum of a mass percentage of the first liquid crystal monomer and mass percentages of all the liquid crystal compounds in the second liquid crystal monomer is 100%. A total mass of the first liquid crystal monomer and the second liquid crystal monomer is defined as 100 parts by weight. Then, a mass of the additive is 1 part by weight.

The formulation of the liquid crystal composition is shown in Table 5. The components in Table 5 are added to a sample vial with a magnetic stir bar, heated and stirred until clear, and cooled down to room temperature, to prepare the liquid crystal composition.

**Table 5**

| Component | Parts by mass |
|---|---|
| 3UTGTS5 | 6.0 |
| 3CP02 | 3.0 |
| 2PTGF | 3.0 |
| 5PTPO2 | 5.0 |
| 2PTPO1 | 3.0 |
| 3PTPO1 | 3.0 |
| 5PTPO1 | 3.0 |
| 3PGTP2 | 5.0 |
| 4UTPP3 | 10.0 |
| 3UTPP4 | 10.0 |
| 3UTPP2 | 5.0 |
| 3UTPP2 | 5.0 |
| 3CCGUF | 3.0 |
| 5CCPUF | 3.0 |
| 3PPGGF | 3.0 |
| 2UTGTP5 | 6.0 |
| 3UTGTP5 | 6.0 |
| 3UTP(1)TP3 | 6.0 |
| 4UTP(1)TP3 | 6.0 |
| 4UTP(1)TP2 | 6.0 |
| UV-P | 1.0 |

The liquid crystal composition provided in Embodiment 5 exhibits good mutual miscibility among the components. Through testing, the liquid crystal composition provided in Embodiment 5 has a clearing point (Cp) of 147°C and birefringence △n of 0.3659 at 25°C and at 589 nm.

### Embodiment 6

Embodiment 6 provides a liquid crystal composition. The liquid crystal composition includes a first liquid crystal monomer and a second liquid crystal monomer. The first liquid crystal monomer is the compound 4UTP(1)S3 prepared in Embodiment 3. The second liquid crystal monomer includes a plurality of liquid crystal compounds, and chemical structures of these liquid crystal compounds can be deduced based on names of corresponding codes in Table 6.

A sum of a mass percentage of the first liquid crystal monomer and mass percentages of all the liquid crystal compounds in the second liquid crystal monomer is 100%. A total mass of the first liquid crystal monomer and the second liquid crystal monomer is defined as 100 parts by weight.

The formulation of the liquid crystal composition is shown in Table 6. The components in Table 6 are added to a sample vial with a magnetic stir bar, heated and stirred until clear, and cooled down to room temperature, to prepare the liquid crystal composition.

**Table 6**

| Component | Parts by mass |
|---|---|
| 4UTP(1)S3 | 6.0 |
| 3CP02 | 3.0 |
| 2PTGF | 3.0 |
| 5PTPO2 | 5.0 |
| 2PTPO1 | 3.0 |
| 3PTPO1 | 3.0 |
| 5PTPO1 | 3.0 |
| 3PGTP2 | 5.0 |
| 4UTPP3 | 10.0 |
| 3UTPP4 | 10.0 |
| 3UTPP2 | 5.0 |
| 3UTPP2 | 5.0 |
| 3CCGUF | 3.0 |
| 5CCPUF | 3.0 |
| 3PPGGF | 3.0 |
| 2UTGTP5 | 6.0 |
| 3UTGTP5 | 6.0 |
| 3UTP(1)TP3 | 6.0 |
| 4UTP(1)TP3 | 6.0 |
| 4UTP(1)TP2 | 6.0 |

The liquid crystal composition provided in Embodiment 6 exhibits good mutual miscibility among the components. Through testing, the liquid crystal composition provided in Embodiment 6 has a clearing point (Cp) of 131°C and birefringence △n of 0.3399 at 25°C and at 589 nm.

### Comparative Example 1

Comparative Example 1 provides a liquid crystal composition. The liquid crystal composition includes a plurality of liquid crystal compounds and an additive. Chemical structures of these liquid crystal compounds can be deduced based on names of corresponding codes in Table 7.

The formulation of the liquid crystal composition in Comparative Example 1 is shown in Table 7. The components in Table 7 are added to a sample vial with a magnetic stir bar, heated and stirred until clear, and cooled down to room temperature, to prepare the liquid crystal composition.

**Table 7**

| Component | Parts by mass |
|---|---|
| 3PTGNCS | 8.0 |
| 5PTGNCS | 24.0 |
| 7PTGNCS | 17.6 |
| 2CPTGNCS | 7.2 |
| 4CPTGNCS | 11.2 |
| 3PPUNCS | 6.0 |
| 5PPUNCS | 4.4 |
| 5PPTP(2)TGNCS | 4.0 |
| 5PPTP(2)TP(2)NCS | 4.0 |
| 5PPTP(2)TVNCS | 4.0 |
| 5PPTP(2)TPNCS | 4.0 |
| O5MTP(2)TPNCS | 5.6 |

Through testing, the liquid crystal composition provided in Comparative Example 1 has a clearing point (Cp) of 140°C and birefringence △n of 0.4440 at 25°C and at 589 nm.

### Comparative Example 2

Comparative Example 2 provides a liquid crystal composition. The liquid crystal composition includes a plurality of liquid crystal compounds and an additive. Chemical structures of these liquid crystal compounds can be deduced based on names of corresponding codes in Table 8.

The formulation of the liquid crystal composition in Comparative Example 2 is shown in Table 8. The components in Table 8 are added to a sample vial with a magnetic stir bar, heated and stirred until clear, and cooled down to room temperature, to prepare the liquid crystal composition.

**Table 8**

| Component | Parts by mass |
|---|---|
| 3CPO2 | 5.0 |
| 4PTPO2 | 4.0 |
| 3PTPO2 | 4.0 |
| 3PTGF | 4.0 |
| 5UTPP3 | 8.0 |
| 4UTPP3 | 8.0 |
| 3UTPP2 | 8.0 |
| 3UTPP4 | 8.0 |
| 2UTPP3 | 8.0 |
| 2UTPP4 | 8.0 |
| 5PGGF | 5.0 |
| 3UTGTP2 | 5.0 |
| 4UTGTP5 | 5.0 |
| 4UTGTP5 | 5.0 |
| 3UTP(1)TP2 | 5.0 |
| 4UTP(1)TP2 | 5.0 |
| 4UTP(1)TP3 | 5.0 |

Through testing, the liquid crystal composition provided in Comparative Example 2 has a clearing point (Cp) of 134°C and birefringence △n of 0.3569 at 25°C and at 589 nm.

### Test Example

In this test example, related performance of the liquid crystal compositions provided in Embodiment 4, Embodiment 5, Embodiment 6, Comparative Example 1, and Comparative Example 2 is tested separately. Testing results are shown in Table 9 to Table 11.

**Table 9**

| Project | Embodiment 4 | Embodiment 5 | Embodiment 6 | Comparative Example 1 | Comparative Example 2 |
|---|---|---|---|---|---|
| Result obtained after 20 days of storage at -30°C | No crystallization | No crystallization | No crystallization | Crystallization | Crystallization |
| Low-temperature stability | Excellent | Excellent | Excellent | Poor | Poor |

**Table 10**

| Project | Embodiment 4 | Embodiment 5 | Embodiment 6 | Comparative Example 1 | Comparative Example 2 |
|---|---|---|---|---|---|
| Color change | Colorless and transparent | Slightly yellow | Slightly yellow | Yellow | Yellow |
| UV resistance capability | Excellent | Good | Good | Poor | Poor |

**Table 11**

| Project | Embodiment 4 | Embodiment 5 | Embodiment 6 | Comparative Example 1 | Comparative Example 2 |
|---|---|---|---|---|---|
| Color change | Colorless and transparent | Colorless and transparent | Colorless and transparent | Yellow | Yellow |
| High-temperature stability | Excellent | Excellent | Excellent | Poor | Poor |

In conclusion, the compound I provided in embodiments of the present disclosure is used as the first liquid crystal monomer, and the first liquid crystal monomer, the second liquid crystal monomer, and an optional additive produce synergistic effect. Therefore, the liquid crystal compositions prepared in Embodiment 4, Embodiment 5, and Embodiment 6 exhibit the following beneficial effects: high birefringence, strong low-temperature stability, strong high-temperature stability, an excellent ultraviolet resistance capability, and a wide nematic-phase temperature range.

The foregoing descriptions are merely intended to help a person skilled in the art understand the technical solutions of the present disclosure, but are not intended to limit the present disclosure. Any modification, equivalent replacement, or improvement made without departing from the spirit and principle of the present disclosure shall fall within the protection scope of the present disclosure.

## Claims

1. A compound, wherein the compound comprises a first chain hydrocarbon group, a first phenyl group, a second phenyl group, a thieno[3,2-b]thiophene group, and a second chain hydrocarbon group;
the first chain hydrocarbon group is linked to one carbon atom of the first phenyl group, another carbon atom of the first phenyl group is linked to one carbon atom of the second phenyl group by a first linkage bond, another carbon atom of the second phenyl group is linked to one carbon atom of the thieno[3,2-b]thiophene group by a second linkage group, and another carbon atom of the thieno[3,2-b]thiophene group is linked to the second chain hydrocarbon group;
the first phenyl group has one or two first substituents, the first substituent is adjacent to the carbon atom that is in the first phenyl group and that is linked to the first linkage bond, and the first substituent is F, -CF₃, or -OCF₃; and
the first chain hydrocarbon group and the second chain hydrocarbon group are selected from linear alkyl, linear alkoxy, linear fluoroalkyl, linear alkenyl, linear alkenyloxy, or difluorovinyl.

2. The compound according to claim 1, wherein the first chain hydrocarbon group and the second chain hydrocarbon group are each independently selected from the linear alkyl with a number of carbon atoms of 1 to 9, the linear alkoxy with a number of carbon atoms of 1 to 9, the linear fluoroalkyl with a number of carbon atoms of 1 to 9, the linear alkenyl with a number of carbon atoms of 2 to 9, the linear alkenyloxy with a number of carbon atoms of 2 to 9, or the difluorovinyl with a number of carbon atoms of 2 to 9.

3. The compound according to claim 1 or 2, wherein the second phenyl group is substituted or unsubstituted; and the substituted second phenyl group has one or more second substituents, and the second substituent is selected from -F, -CH₃, -CH₂CH₃, -OCF₃, cyclopropyl, cyclobutyl, or cyclohexyl.

4. The compound according to any one of claims 1 to 3, wherein the first linkage bond and the second linkage bond are each independently selected from a carbon-carbon single bond, a carbon-carbon double bond, an alkynyl bond, an ester bond, or a difluoromethyl ether bridge bond.

5. The compound according to any one of claims 1 to 4, wherein a chemical structural formula of the compound is shown as follows: 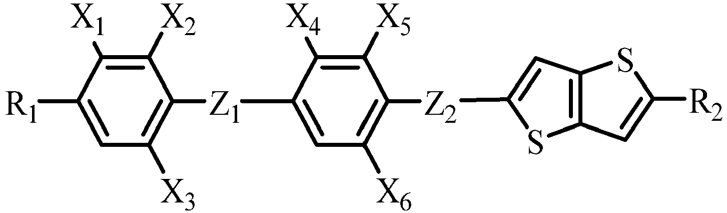
wherein R₁ and R₂ respectively represent the first chain hydrocarbon group and the second chain hydrocarbon group, and the first chain hydrocarbon group and the second chain hydrocarbon group are each independently selected from the linear alkyl with a number of carbon atoms of 1 to 9, the linear alkoxy with a number of carbon atoms of 1 to 9, the linear fluoroalkyl with a number of carbon atoms of 1 to 9, the linear alkenyl with a number of carbon atoms of 2 to 9, the linear alkenyloxy with a number of carbon atoms of 2 to 9, or the difluorovinyl with a number of carbon atoms of 2 to 9;
Z₁ and Z₂ respectively represent the first linkage bond and the second linkage bond, and the first linkage bond and the second linkage bond are each independently selected from and independently represent a carbon-carbon single bond, a carbon-carbon double bond, alkynyl, ester, or a difluoromethyl ether bridge bond;
X₁ and X₄ to X₆ are each independently selected from -H, -F, -CH₃, -CH₂CH₃, -OCF₃, cyclopropyl, cyclobutyl, or cyclohexyl; and
at least one of X₂ and X₃ is F, -CF₃, or -OCF₃.

6. The compound according to claim 5, wherein the compound is a compound I-1, a compound I-2, a compound I-3, or a compound I-4; and
a chemical structural formula I-1 corresponding to the compound I-1, a chemical structural formula I-2 corresponding to the compound I-2, a chemical structural formula I-3 corresponding to the compound I-3, and a chemical structural formula I-4 corresponding to the compound I-4 are separately shown as follows: 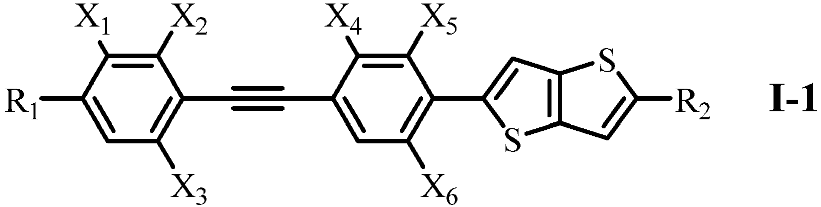 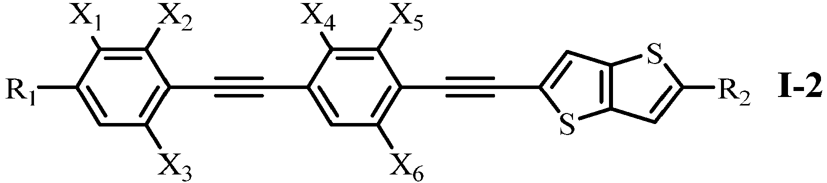 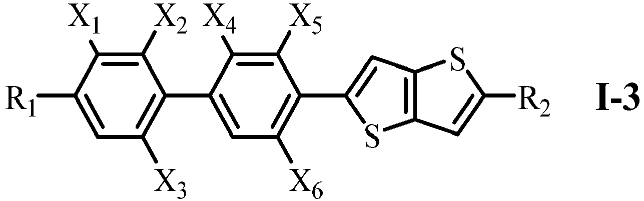 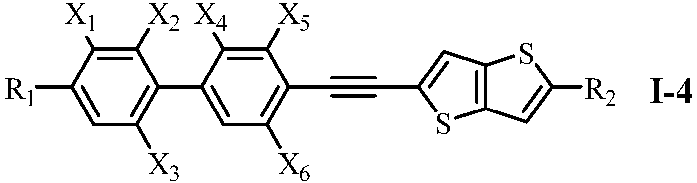
wherein R₁ and R₂ are each independently selected from the linear alkyl with the number of carbon atoms of 1 to 9 or the linear alkoxy with the number of carbon atoms of 1 to 9.

7. Use of the compound according to any one of claims 1 to 6 in a liquid crystal material.

8. A method for preparing the compound according to any one of claims 1 to 6, wherein the preparation method comprises:
determining, based on a chemical structure of the compound, a plurality of monomers for synthesizing the compound; and
subjecting the plurality of monomers to a reaction step by step to prepare the compound, wherein
the reaction comprises at least one of a Sonogashira coupling reaction, a Suzuki coupling reaction, and a halogenation reaction.

9. A liquid crystal material, wherein the liquid crystal material comprises a first liquid crystal monomer and a second liquid crystal monomer, and the first liquid crystal monomer comprises the compound according to any one of claims 1 to 6; and
the second liquid crystal monomer is configured to ensure that there is no crystallization phenomenon at a temperature less than or equal to 0°C after the second liquid crystal monomer and the first liquid crystal monomer are mixed.

10. The liquid crystal material according to claim 9, wherein the second liquid crystal monomer is selected from at least one of a compound II-1, a compound II-2, a compound II-3, a compound II-4, a compound II-5, a compound II-6, a compound II-7, and a compound II-8; and
a chemical structural formula II-1 corresponding to the compound II-1, a chemical structural formula II-2 corresponding to the compound II-2, a chemical structural formula II-3 corresponding to the compound II-3, a chemical structural formula II-4 corresponding to the compound II-4, a chemical structural formula II-5 corresponding to the compound II-5, a chemical structural formula II-6 corresponding to the compound II-6, a chemical structural formula II-7 corresponding to the compound II-7, and a chemical structural formula II-8 corresponding to the compound II-8 are separately shown as follows: 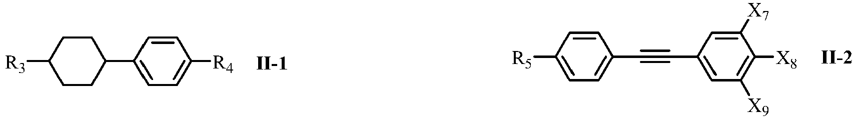 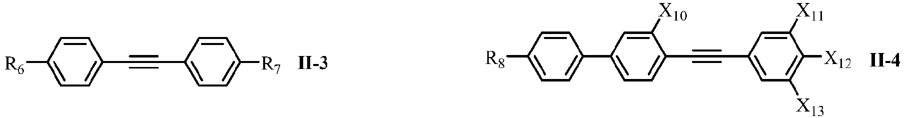 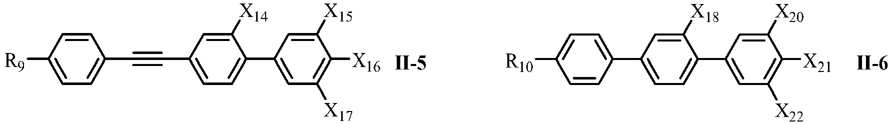 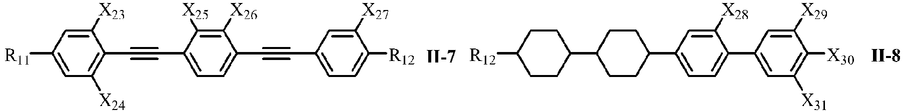
wherein R₃ to R₁₂ are each independently selected from one of linear alkyl with a number of carbon atoms of 1 to 6 and linear alkoxy with a number of carbon atoms of 1 to 6; and
X₇ to X₃₁ are each independently selected from -H, -F, -CH₃, -CH₂CH₃, or -OCF₃.

11. The liquid crystal material according to claim 9 or 10, wherein the liquid crystal material further comprises an additive, and the additive is selected from at least one of an ultraviolet absorber, an ultraviolet stabilizer, and an antioxidant.

12. The liquid crystal material according to claim 9 or 10, wherein the liquid crystal material has at least one of the following characteristics: no crystallization at -60°C to 0°C; no color change at 0°C to 150°C; and no color change at a UV intensity less than or equal to 80 mW/cm².

13. An optoelectronic device, wherein the optoelectronic device comprises an encapsulation structure and a liquid crystal material encapsulated in the encapsulation structure, and the liquid crystal material is the liquid crystal material according to any one of claims 9 to 12.

14. The optoelectronic device according to claim 13, wherein the optoelectronic device comprises a wavelength selective switch, a microwave antenna, or a display device.
